(19) Europäisches Patentamt
European Patent Office
Office européen des brevets

(11) **EP 2 671 511 B1**

(12) **EUROPEAN PATENT SPECIFICATION**

(45) Date of publication and mention
of the grant of the patent:
**08.11.2017 Bulletin 2017/45**

(51) Int Cl.:
***A61B 8/08*** (2006.01)

(21) Application number: **11857846.7**

(22) Date of filing: **28.12.2011**

(86) International application number:
**PCT/JP2011/080448**

(87) International publication number:
**WO 2012/105152 (09.08.2012 Gazette 2012/32)**

(54) **ULTRASOUND DIAGNOSTIC APPARATUS AND METHOD**

ULTRASCHALL-DIAGNOSEVORRICHTUNG UND VERFAHREN

APPAREIL ET PROCÉDÉ DE DIAGNOSTIC À ULTRASONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.02.2011 JP 2011023152**

(43) Date of publication of application:
**11.12.2013 Bulletin 2013/50**

(73) Proprietor: **Hitachi, Ltd.
Chiyoda-ku
Tokyo 100-8280 (JP)**

(72) Inventors:
• **TABARU, Marie
Chiyoda-ku, Tokyo 100-8280 (JP)**

• **AZUMA ,Takashi
Chiyoda-ku, Tokyo 100-8280 (JP)**
• **YOSHIKAWA, Hideki
Chiyoda-ku, Tokyo 100-8280 (JP)**
• **HASHIBA, Kunio
Chiyoda-ku, Tokyo 100-8280 (JP)**

(74) Representative: **Strehl Schübel-Hopf & Partner
Maximilianstrasse 54
80538 München (DE)**

(56) References cited:
**JP-A- 2003 210 460     JP-A- 2007 105 400
JP-A- 2009 201 989     US-A1- 2009 216 119
US-A1- 2010 016 718     US-A1- 2010 016 718**

**Description**

The present invention relates to an ultrasound diagnostic apparatus which diagnoses a test body by sending and receiving ultrasonic waves, and more particularly, the present invention relates to an ultrasound diagnostic technique which detects the difference in stiffness inside the test body.

Background Art

There has been employed a method of diagnosing the stiffness (strain, elastic modulus and the like) inside the test body based on an ultrasonic echo signal (elastography technique) as a diagnostic method of breast cancer, liver cirrhosis, and angiopathy of a living body as the test body instead of palpation given by a doctor. When diagnosing the stiffness using the elastography technique, the practitioner tightly contacts an ultrasound probe with the test body surface so as to generate displacement in the tissue inside the body (this method will be referred to as a general method). Based on the echo signals before and after the pressure contact of the tissue of the body, the displacement in the compression direction is estimated so that the strain as the spatial differentiating amount of the displacement is obtained and imaged. This method is extremely effective for the substance to be imaged, that is, the organ (for example, mammary gland) that exists at the position which can be easily pressed from the body surface. However, it is not effective for all the substances to be imaged. For example, a slip plane exists as an intervening layer between the body surface and the liver, and it is difficult to apply the pressure to generate sufficient displacement. Estimation of the elastic modulus using the general method requires boundary conditions. In this case, however, the slip plane may make the boundary conditions more complicated, resulting in difficult estimation of the elastic modulus.

Alternatively, the technique of diagnosing the stiffness has been introduced by using the focused beam as a displacement generating transmission beam for applying the radiation pressure to the inside of the test body, and displacing the target tissue while suppressing the influence of the intervening layer. Patent Literature 1 discloses ARFI (Acoustic Radiation Force Impulse) imaging, for example. The technique images the displacement amount of the tissue generated in the moving direction of the focused beam, and measures and images the elastic modulus as the average shear elastic modulus (hereinafter referred to as the elastic modulus) in the shear wave propagation region based on the estimation of the propagation speed of the shear wave generated in the direction vertical to the advancing direction of the focused beam in association with the tissue displacement at the focal point. Use of the elastic modulus measurement technique through generation of the shear wave provides the effect of allowing the ultrasonic waves to displace the tissue in addition to reduction of the influence of the intervening layer such as the slip plane as described above. This technique is expected to allow the diagnosis to be less dependent on the procedure.

Patent Literature 2 discloses the method capable of imaging the dense elastic modulus distribution by measuring the elastic modulus at a plurality of positions within the image pickup field, and correlating the dense strain distribution (relative value of stiffness) derived from the general method to the rough elastic modulus (absolute value of stiffness). Generally, the term "absolute value" may refer to an unsigned value indicating the distance from 0, or the term as an antonym of the "relative value". The term "absolute value" herein will be mainly used as the antonym of the term "relative value".

Citation List

Patent Literature

Patent Literature 1: Publication of US patent application No. 2004/068184
Patent Literature 2: JP-A-2009-201989 (which is a family member of US 2009/216119 A1)

Summary of Invention

Technical Problem

Generally, attenuation of the shear wave is larger than that of the longitudinal wave. When irradiating the single focal point with the focused beam for measuring the elastic modulus by the shear wave generation at the single point, the region where the elastic modulus can be measured is limited to be in the range of the shear wave propagation distance (approximately 5 to 10 mm). When measuring the elastic modulus in the wider region, the elastic modulus has to be measured by generating the shear waves at a plurality of points inside the desired region. Meanwhile, when measuring the elastic modulus by generating the shear wave at each point, the focused beam radiation time (several

hundreds μs to 1 ms) is longer than the ultrasonic beam radiation time used for a B mode image by several hundreds to several thousands times. The temperature rise of the body tissue and the ultrasound probe is proportional to the radiation time. Compared with measurement of the B mode image, safety with respect to the temperature rise may be deteriorated. The time interval of the elastic modulus measurement may be increased (by 1 to 2 seconds) sufficiently for the purpose of safely measuring the elastic modulus while suppressing the temperature rise in the wider region. Increase in the time interval of the elastic modulus measurement reduces the frame rate (the number of updatings of the screen per unit of time). If the frame rate is reduced, the influence of the body motion of the test body becomes significant. This may deteriorate accuracy of the elastic modulus measurement owing to shifting of the fault planes and the strain owing to compression. As described above, measurement of the elastic modulus by generating the shear wave requires the technique that allows measurement of the elastic modulus in the wider range while suppressing decrease in the frame rate so as to ensure safety.

The technique disclosed in Patent Literature 2 may improve safety, and ensures measurement of the elastic modulus over a wide range. However, Patent Literature 2 does not disclose the method of selecting the position at which the elastic modulus is measured (the position at which the shear wave is generated). If a plurality of stiffness parts exists in the region at the single position where the elastic modulus is measured, the measured elastic modulus may be provided as the average value, thus deteriorating the measurement accuracy. The number of elastic modulus measurement positions has to be increased for improving the measurement accuracy. The increase in the measurement points may deteriorate the frame rate. As described above, the elastic modulus has to be measured the number of times as small as possible by selecting the position optimal for the elastic modulus measurement with high accuracy.

In view of the aforementioned problems, it is an object of the present invention to provide an ultrasound diagnostic apparatus and an ultrasound display method capable of improving accuracy of the elastic modulus measurement by preliminarily selecting the position optimal for measuring the elastic modulus of the test body so as to measure the elastic modulus.

US 2010/016718 A1 discloses an apparatus in accordance with the preamble of present claim 1. Means for solving the problem

In order to achieve the object, the present invention provides an ultrasound diagnostic apparatus as defined in claim 1 and a method as defined in claim 10. The subclaims relate to preferred embodiments.

Advantageous Effects of Invention

The present invention provides the ultrasound diagnostic apparatus with high accuracy and a method of hybrid type for displaying a combined image of the strain image generated based on the strain information and the elastic modulus derived from the shear wave generation. The single position is set as the elastic modulus measurement point through generation of the shear wave. This makes it possible to provide the significantly safe ultrasound diagnostic device and the ultrasound display method. Brief Description of Drawings

FIG. 1 is a view showing an overall system structure of an ultrasound diagnostic apparatus according to a first example.
FIG. 2 is an explanatory view showing a method of displaying a strain image based on strain information according to the first example.
FIG. 3 is a view showing a flowchart of the process executed using a hybrid method according to the first example.
FIGS. 4A and 4B are explanatory views showing a method of selecting an elastic modulus measurement position according to the first example.
FIGS. 5A and 5B are explanatory views showing an ultrasonic beam forming according to the first example.
FIG. 6 is a view showing a measurement performed by an ultrasound probe according to the first example.
FIG. 7 is an explanatory view showing a method of estimating a shear wave propagation speed according to the first example.
FIG. 8 is a view showing a relationship between the elastic modulus and the strain.
FIG. 9 is an explanatory view showing a method of displaying the elastic modulus image according to the first example.
FIG. 10 is a view showing an overall system structure of the ultrasound diagnostic apparatus according to a second example.
FIG. 11 is a view showing a measurement using the ultrasound probe according to the second example.
FIG. 12 is an explanatory view showing a sequence of sending and receiving ultrasound waves according to the second example.
FIG. 13 is a view showing a shear wave penetration according to the second example.
FIG. 14 is an explanatory view showing a direction of the shear wave displacement and a direction of the shear wave propagation according to the second example.

FIG. 15 is an explanatory view showing a relationship of a distance between two focal points, and a temperature rise according to the second example.

FIGS. 16A-16D are conceptual views showing an emphasis on sensitivity and safety derived from a burst-chirp method according to the second example.

FIG. 17 is a flowchart of the process executed using the hybrid method according to the second example.

FIG. 18 is an explanatory view showing a selected elastic modulus measurement position according to a fourth example.

FIGS. 19A and 19B are explanatory views showing each size of a kernel and a filter according to a fifth example.

FIG. 20 is a view showing a relationship between the elastic modulus and the strain according to the fifth example.

FIG. 21 is a view showing an overall structure of an ultrasound diagnostic system according to a first modified example of the first example.

FIGS. 22A and 22B are explanatory views showing radiation of displacement generating ultrasound waves in the ultrasound diagnostic apparatus according to a second modified example of the first example.

FIG. 23 is a view showing an overall structure of the ultrasound diagnostic system according to the second modified example of the first example.

Description of Embodiment

Examples of an embodiment according to the present invention will be described. Hereinafter, various functional programs executed by the processing part of the computer will be expressed by functions, means and units. For example, the program for computing the elastic modulus will be referred to as an elastic modulus computing function, elastic modulus computing means, an elastic modulus computing unit, or the like. As described above, the process of combining the strain image based on strain information and the elastic modulus measured through shear wave generation will be referred to as the hybrid method.

First example

A first example relates to an ultrasound diagnostic apparatus using an ultrasound probe for transmitting an ultrasonic beam to a test body and receiving an echo signal, which includes a strain computing unit 24 which radiates a first displacement detection beam, and computes strain information in a region 1 based on the echo signal from the test body that receives the first displacement detection beam, a displacement generating unit 10 which radiates a focused beam to an inside of the test body to displace a tissue in the test body, an elastic modulus computing unit 34 which radiates a second displacement detection beam, and detects a shear wave displacement generated by the focused beam based on the echo signal from the test body that receives the second displacement detection beam for detecting an elastic modulus in a region 2 included in the region 1, and a display unit 7 which displays a strain image based on the strain information and the elastic modulus.

The ultrasound diagnostic apparatus of this example includes a measurement position selecting unit 40 which selects at least one elastic modulus detection position at which the elastic modulus is detected based on the strain information. At least one focal point position irradiated by the focused beam is determined from at least the one elastic modulus detection unit selected by the measurement position selecting unit 40. The measurement position selecting unit 40 allows the operator to select the elastic modulus detection position while observing the image displayed on the display unit 7. The display unit 7 displays the color scale indicating the elastic modulus in the display range of the strain image to be displayed.

The example relates to an ultrasound diagnostic apparatus using an ultrasound probe for transmitting an ultrasonic beam to a test body and receiving an echo signal, which includes a strain computing unit 24 which radiates a first displacement detection beam, and computes strain information in a region 1 based on the echo signal from the test body that receives the first displacement detection beam, a measurement position selecting unit 40 which selects a region 2 included in the region 1 based on the strain information, a displacement generating unit 10 which displaces a tissue inside the test body by radiating a focused beam to the inside of the test body, and an elastic modulus computing unit 34 which radiates a second displacement detection beam, and detects a shear wave displacement generated by the focused beam based on the received echo signal from the test body so as to detect the elastic modulus in the region 2.

In the ultrasound diagnostic apparatus as described above, the measurement position selecting unit 40 obtains a position at which a standard deviation of a strain distribution in the region 1 or a difference between a maximum value and a minimum value is smaller than a threshold value when selecting the region 2. The elastic modulus computing unit 34 calculates a stress using the elastic modulus in the region 2 and the strain information in the region 2, and computes the elastic modulus of the region 1 from the strain information in the region 1 and the stress. The measurement position selecting unit 40 selects the region 2 by extracting a contour of the strain distribution in the region 1 by processing the image. The ultrasound diagnostic apparatus further includes a display unit 7. The measurement position selecting unit 40 allows an operator to select the region 2 based on the strain image displayed on the display unit 7.

The example relates to an ultrasound display method of displaying an image on a display unit using an ultrasound probe for transmitting an ultrasound beam to a test body and receiving an echo signal from the test body based on the received echo signal. The method includes the steps of computing strain information in a first region by radiating a first displacement detection beam and receiving the echo signal from the test body, displaying a strain image based on the computed strain information on the display unit, displacing a tissue of the test body by radiating a focused beam in the test body; radiating the second displacement detection beam and receiving the echo signal from the test body to detect a shear wave displacement generated by the focused beam, computing an elastic modulus in the second region included in the first region based on the shear wave displacement, and the computed elastic modulus is displayed on the display unit 7.

The example provides the aforementioned ultrasound diagnostic method for determining the focal point position of the focused beam selected based on the strain information. The example also relates to the ultrasound diagnostic method in which the second region irradiated by the second displacement detection beam is selected from a location with the uniform strain information based on the strain image displayed on the display unit. In the ultrasound diagnostic method, the display unit displays a scale indicating the elastic modulus in a display range of the strain image to be displaced.

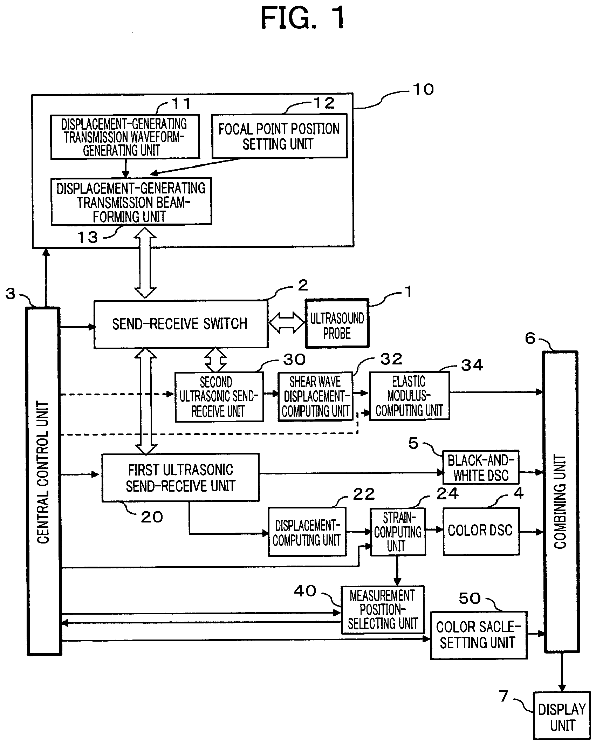

FIG. 1 shows a specific example of the overall system structure of the ultrasound diagnostic apparatus according to a first example. An ultrasound probe 1 brought into contact with an outer skin of a not shown test body includes an ultrasound send-receive surface having an arrangement of a plurality of oscillators for sending and receiving ultrasonic waves to and from the test body, and is connected to a send-receive switch 2. A central control unit 3 serves to control the ultrasound diagnostic apparatus. Especially, the central control unit 3 is configured to control a displacement generating unit 10 for generating the displacement in the test body, the send-receive switch 2, a first ultrasonic send-receive unit 20, a strain computing unit 24, a second ultrasonic send-receive unit 30, an elastic modulus computing unit 34 for computing the elastic modulus, a measurement position selecting unit 40, and a color scale setting unit 50. The ultrasound probe 1 is connected to a displacement generating transmission beam forming unit 13, the first ultrasonic send-receive unit 20, and the second ultrasonic send-receive unit 30 via the send-receive switch 2.

The send-receive switch 2 is controlled to interrupt or cut the connection between the ultrasound probe 1 and the displacement generating transmission beam forming unit 13, the first ultrasonic send-receive unit 20, and the second ultrasonic send-receive unit 30 via the central control unit 3. The displacement generating transmission beam forming unit 13 generates the focused beam radiated to the inside of the test body so as to displace the tissue inside the test body.

The first ultrasonic send-receive unit 20 is controlled by the central control unit 3 to add the delay time and weight to a wave transmission signal of an element of the ultrasound probe 1 using the waveform generated by a displacement detection transmission waveform generating unit which is not shown in the drawing so that the displacement detection ultrasonic beam is focused at a desired position of the not shown test body. An echo signal reflected by the inside of the test body to return to the probe is converted into an electric signal by the ultrasound probe 1, and is sent to the first ultrasonic send-receive unit 20. The first ultrasonic send-receive unit 20 includes a signal processing circuit which performs phasing addition of the echo signal, envelope demodulation, log compression, band pass filter, and gain control.

An output signal from the first ultrasonic send-receive unit 20 is input to a black-and-white DSC (Digital Scan Converter) 5, and a displacement computing unit 22. The black-and-white DSC 5 generates fault layer image (B mode image) information indicating luminance of black and white. The displacement computing unit 22 is configured to compute the displacement of each point by an image correlation using the fault images of two adjacent frames. The displacement information output from the displacement computing unit 22 is input to a strain computing unit 24 so that only the strain at each point is computed based on a displacement spatial differentiation. The strain information is input to the color DSC 4 which provides hue modulation in accordance with the value of the strain information.

An image 41 having hue modulated by the color DSC 4 (hereinafter referred to as a strain image) based on the strain information is sent to a combining unit 6, which is combined with a B mode image 45. The combined image is then displayed on the display unit 7 as the strain image. The strain information calculated by the strain computing unit 24 is sent to a measurement position selecting unit 40 and a color scale setting unit 50 via the central control unit 3. The color scale setting unit 50 generates a color scale 43 corresponding to the strain image 41 based on maximum and minimum values of the strain.

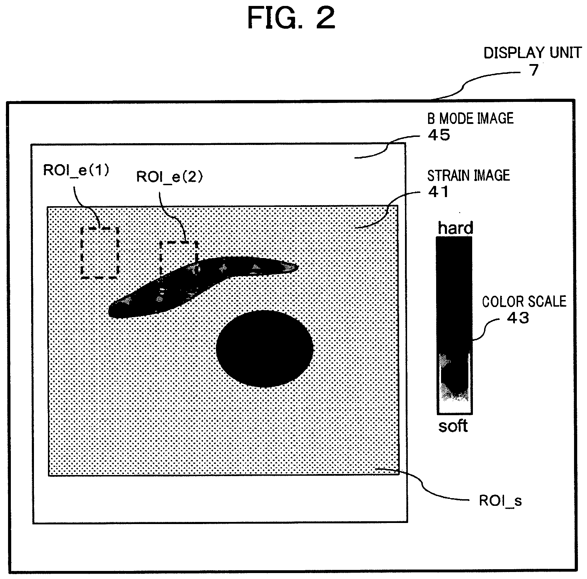

Referring to FIG. 2, the display unit 7 displays the color scale 43 adjacent to the B mode image 45 and the strain image 41. Referring to the drawing, such terms as "hard" indicating a small strain, and "soft" indicating a large strain are marked adjacent to the color scale. The strain image 41 has been described as the image having hue modulated based on the strain information. However, it is possible to provide the monochrome modulated image based on the strain information.

The strain information calculated by the strain computing unit 24 is sent to the measurement position selecting unit 40. The measurement position selecting unit 40 performs the signal processing based on two-dimensional strain information, and determines the position in the fault plane where the elastic modulus is measured. The position information determined by the measurement position selecting unit 40 is input to a focal point position setting unit 12 of the displacement generating unit 10 via the central control unit 3.

The displacement generating unit 10 will be described. The displacement generating transmission beam forming unit 13 for forming the focused beam is controlled by the central control unit 3 to add the delay time and weight to the wave transmission signal of an element 100 of the ultrasound probe 1 using the waveform generated by the displacement generating transmission waveform generating unit 11 so that the ultrasonic beam is focused on the position set by the focal point position setting unit 12, that is, the focused position set based on the position determined by the measurement position selecting unit 40. The electric signal from the displacement generating transmission beam forming unit 13 is converted into an ultrasonic signal by the ultrasound probe 1 via the send-receive switch 2. Then the focused beam for displacement generation is radiated to the not shown test body.

The second ultrasonic send-receive unit 30 is controlled by the central control unit 3 to add the delay time and weight to the wave transmission signal of the element of the ultrasound probe 1 using the waveform generated by the displacement detection transmission waveform generating unit not shown in the drawing so that the displacement detection ultrasonic beam is focused on the desired position of the not shown test body. The echo signal reflected by the inside of the test body to return to the probe is converted into an electric signal by the ultrasound probe 1, and is sent to the second ultrasonic send-receive unit 30. The second ultrasonic send-receive unit 30 includes a signal processing circuit which performs phasing addition of the echo signal, envelope demodulation, log compression, band pass filter, and gain control.

An output signal from the second ultrasonic send-receive unit 30 is input to a shear wave displacement computing unit 32. The shear wave displacement computing unit 32 is configured to compute each displacement of the points by correlation computing. The displacement information output from the shear wave displacement computing unit 32 is input to the elastic modulus computing unit 34 so that the value relevant to stiffness such as the propagation speed of the shear wave and elastic modulus is calculated. The value relevant to the stiffness is sent to the combining unit 6 so as to be displayed on the screen together with the B mode image and the strain image displayed by the display unit 7.

The central control unit 3, the displacement computing unit 22, the strain computing unit 24, the shear wave displacement computing unit 32, the elastic modulus computing unit 34, the measurement position selecting unit 40 and the like which constitute a part of the block shown in the drawing may be realized by executing the program by the central processing unit (CPU) serving as the processing unit. The ultrasound diagnostic system according to the first example has been described with respect to the first ultrasonic send-receive unit 20 and the second ultrasonic send-receive unit 30 separately as shown in FIG. 1. Such description has been made by prioritizing the function. From the aspect of mounting the apparatus, it is possible to form the first and the second ultrasonic send-receive unit into the single ultrasonic send-receive unit.

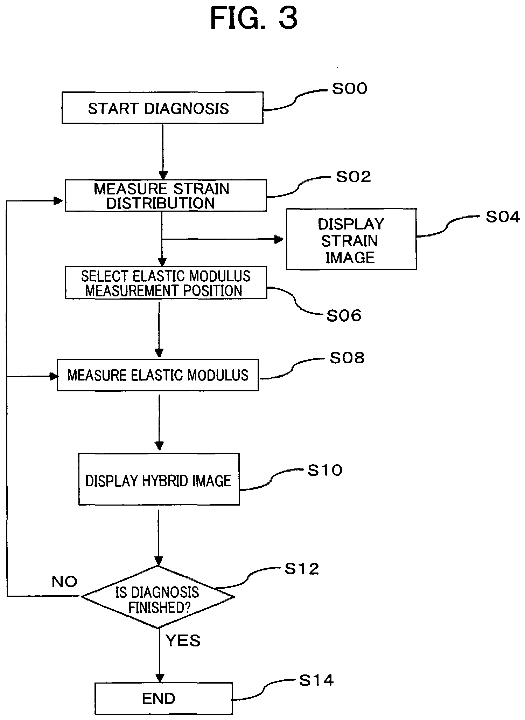

The process flow of the hybrid method of the example will be described referring to FIG. 3. Referring to the process flow shown in FIG. 3, a series of the process will be described sequentially from start to the end of diagnosis subsequent to display of the elastic modulus image. First in step S00, the stiffness diagnosis using the hybrid method is started. The starting signal is input via a not shown input device. Before starting the hybrid method, the B mode image or the strain image is displayed on the display unit 7. Upon start of the diagnosis using the hybrid method, the strain information in a desired region ROI (Region of Interest) input via a not shown input device is obtained (step S02). The ROI for obtaining the strain information will be referred to as ROI_s or a first region (Region 1).

The strain information may be derived from the general method. That is, the ultrasound probe 1 is pressed against the surface of the test body so that the displacement and strain are detected by sending and receiving ultrasonic waves with repetition while compressing the body surface. Upon detection of the strain, the first ultrasonic send-receive unit 20 serves to add the delay time and weight to the ultrasonic transmission, and to convert the echo signal into the electric signal. The displacement computing unit 22 serves to compute the displacement. The strain computing unit 24 serves to calculate the strain information.

When the strain computing unit 24 finishes computing of the strain, the B mode image from the black-and-white DSC 5 and the strain image from the color DSC 4 are combined by the combining unit 6, and the combined image is displayed on the display unit 7 as shown in FIG. 2 (step S04).

At the end of computing the strain, the measurement position selecting unit 40 determines the position optimal for measuring the elastic modulus (step S06). The region where the elastic modulus is measured designated as ROI_e or a second region (Region 2). It is small enough to be included in the region ROI_s or the first region (Region 1) where the strain is displayed. The region optimal for the elastic modulus measurement is the ROI_e(1) shown in FIG. 2, having the same strain value as the one in the ROI_e, that is, the region with the same stiffness. The region having two or more strain amounts mixed in the ROI_e like the RO1_e(2) is not preferable for the elastic modulus measurement. If the elastic modulus is measured in the ROI where two or more strain amounts are mixed, the average elastic modulus is obtained, thus deteriorating the measurement accuracy. It is necessary to set the region having the same value of the strain amount as much as possible (that is, uniform strain amount) as the elastic modulus measurement position. The optimal elastic modulus measurement position will be determined by subjecting the strain information and strain image to the signal processing.

The signal processing using the hybrid method according to the example will be described. In this case, the ultrasound probe 1 of linear array type is brought into contact with the surface of the test body so that the displacement generating transmission beam is focused on the target fault plane in the body. It is assumed that the propagation direction of the displacement generating transmission beam is vertical to the body surface in the desired fault plane.

A rectangular region (kernel K) is produced for conducting the signal processing method. Preferably, the kernel K has the same size as that of the region ROI_e for measuring the elastic modulus, that is, the second region (Region 2). The length of the ROI_e in the depth direction is determined by the width of the focused beam for shear wave generation (=displacement generating transmission beam) upon measurement of the elastic modulus in the depth direction (alternatively, distance direction, displacement direction). The width in the depth direction denotes the value expressed by the width of -6 dB with the beam shape in the depth direction. The length of the ROI_e in the orientation direction is determined by the propagation distance of the shear wave in the propagation direction. In this case, the propagation distance of the shear wave denotes the maximum distance from the focal point position at which the shear wave displacement may be detected in the direction along the shear wave propagation direction. The length of the ROI_e in the depth direction is 10 mm, and the length of the ROI_e in the orientation direction is 5 mm. In such a case, it is preferable to set the length ly of the kernel K in the depth direction to 10 mm, and the length lx of the ROI_e in the orientation direction to 5 mm. Each length of the respective lines of the ROI_e and the kernel K is not limited to 10 mm in the depth direction nor 5 mm in the orientation direction.

The central control unit 3 shown in FIG. 1 reads locations of the mammary gland, prostatic gland, blood vessel and liver, frequency of carrier of the ultrasonic beam for generating the shear wave, F value (= focal point distance/opening aperture), and the size of the kernel K adapted to the focal point distance from a not shown storage medium. The obtained data are employed for the signal processing performed by the measurement position selecting unit 40. The size of the kernel K may be input by the operator via a not shown input medium.

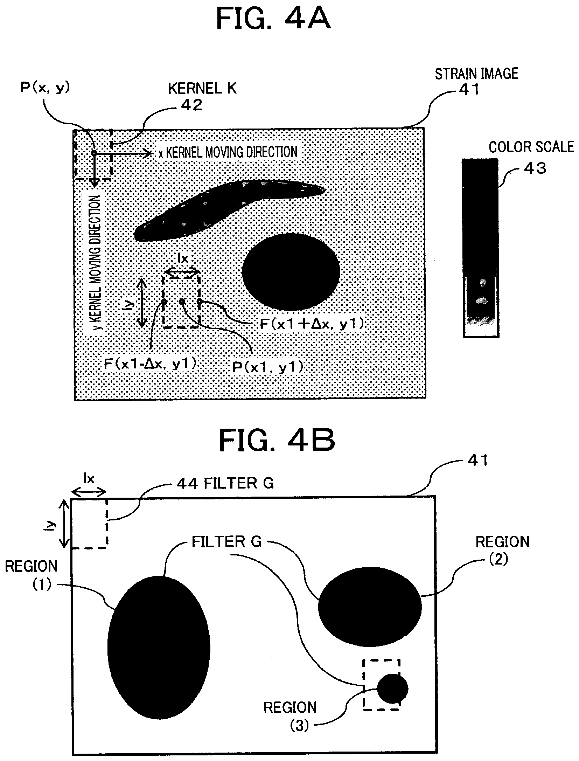

As FIG. 4A shows, a kernel K 42 moves from right to left or up and down in the strain image 41. It is assumed that the position of the kernel K 42 is expressed by P(x,y) as its center position. The x axis denotes the orientation direction, and the y axis denotes the depth direction. Computing is executed using the strain information at each of the positions P(x,y). The optimal elastic modulus measurement position is determined by the value computed using the strain information in the kernel. For example, a standard deviation S(x,y) of the strain in the kernel K is calculated with respect to the kernel position P(x,y). The optimal elastic modulus measurement position is determined as the position at which the standard deviation is minimized. As for another example, the difference between the maximum and minimum values of the strain amount in the kernel, that is, Max-min(x,y) is computed, and the position at which the difference Max-min has the minimum value is determined as the optimal elastic modulus measurement position. Alternatively, the region having the same value of the strain amount may be determined using the known calculation expression.

Besides production of the kernel K 42 for computing as described above, it is possible to apply the image processing to the strain image such as the strain image 41 using the known two-dimensional filter (hereinafter referred to as a filter G) 44 used for the image processing so as to determine the optimal elastic modulus measurement position. Preferably, the filter G 44 has the same size as that of the ROI_e where the elastic modulus is measured likewise the kernel K. For example, the length ly in the depth direction is set to 10 mm, and the length lx in the orientation direction is set to 5 mm. Each length of the lines of the filter G is not limited to 10 mm in the depth direction, nor 5 mm in the orientation direction. The central control unit 3 reads the size of the filter G adapted to locations of the mammary gland, prostatic gland, blood vessel and liver, frequency of carrier of the shear wave generating ultrasonic beam, F value and the focal distance from a not shown storage medium. The obtained information is employed for the signal processing executed by the measurement position selecting unit 40. The size of the filter G may be input by the operator via a not shown input medium.

The filter G 44 is a well-known filter for the image processing technique, for example, and employs a Laplacian filter as the one used for extracting the image contour. The image processing using the Laplacian filter extracts the strain in the strain image 41. Upon extraction of the contour of the strain distribution, the strain information in the strain image 41 is divided into a plurality of regions. As a result of the image processing, a plurality of regions R(1), R(2) and R(3) are obtained as shown in FIG. 4B. For simplification of the description, the number of regions is set to 3. However, the number of the regions may be any integer equal to or larger than 2. Among a plurality of regions, the region R(n) (n=1, 2, 3, ...) is selected as the elastic modulus calculation region ROI_e, that is, the region R(n) larger than the filter G as the optimal elastic modulus measurement region.

Referring to the example shown in FIG. 4B, it is judged that the regions R(1) and R(2) are suitable elastic modulus measurement regions, and the region R(3) is unsuitable elastic modulus measurement region. Among the suitable elastic modulus measurement regions, the optimal region R(n) for the elastic modulus measurement is output to the central control unit 3. In other words, the measurement position selecting unit 40 automatically selects the region with the largest area in the appropriate regions as the optimal region R(n). The filter G is capable of applying the known filter other than the Laplacian filter.

In step S08 of FIG. 3, the focused beam as the displacement generating transmission beam is radiated to the focal point position determined based on the optimal elastic modulus measurement position and the region to generate the shear wave, and the propagation speed of the shear wave is estimated for computing the elastic modulus.

As described above, in this case, the ultrasound probe 1 of linear array type is brought into contact with the test body surface, and the displacement generating transmission beam is focused on the target fault plane in the body. An explanation will be made with respect to the case where the propagation direction of the displacement generating transmission beam is vertically directed to the body surface in the desired fault plane.

Concerning the position of the focal point F of the displacement generating transmission beam, the optimal elastic modulus measurement position determined by the measurement position selecting unit 40 is output to the central control unit 3, through which the position of the focal point F is input to the focal point position setting unit 12.

If the kernel K is produced for calculation so that the optimal elastic modulus measurement position is determined as $P(x1,y1)$ as shown in FIG. 4A, the position of the focal point F is set to $F(x1-\Delta x,y1)$, for example. In this case, the $\Delta x$ is the value corresponding to the value half the width of the ROI_e in the orientation direction, that is, the value corresponding to half of the propagation distance of the shear wave. If the kernel K has the same size as that of the region ROI_e where the elastic modulus is measured, the length of the ROI_e in the orientation direction is lx. Accordingly, the relationship of $\Delta x=lx/2$ is obtained. In such a case, the displacement generating transmission beam is radiated to the left end of the kernel K so as to detect displacement of the shear wave which propagates in +x direction. The displacement direction of the shear wave is vertical to the x axis, that is, y direction.

Assuming that the image processing is executed using the filter G to select the optimal region R(n), and the optimal elastic modulus measurement position is determined as the center of gravity of the R(n), that is, $P(x2,y2)$, the position of the focal point F input to the focal point position setting unit 12 is determined as $P(x2-\Delta x,y2)$.

When converting the optimal elastic modulus measurement position to the position of the focal point F, the central control unit 3 reads an optimal conversion method based on locations of the mammary gland, prostatic gland, blood vessel and liver, carrier frequency of the ultrasonic beam for generating the shear wave, and the F value from a not shown recording medium. Then the central control unit 3 or a not shown central processing unit functioning as the processing unit executes computation for conversion.

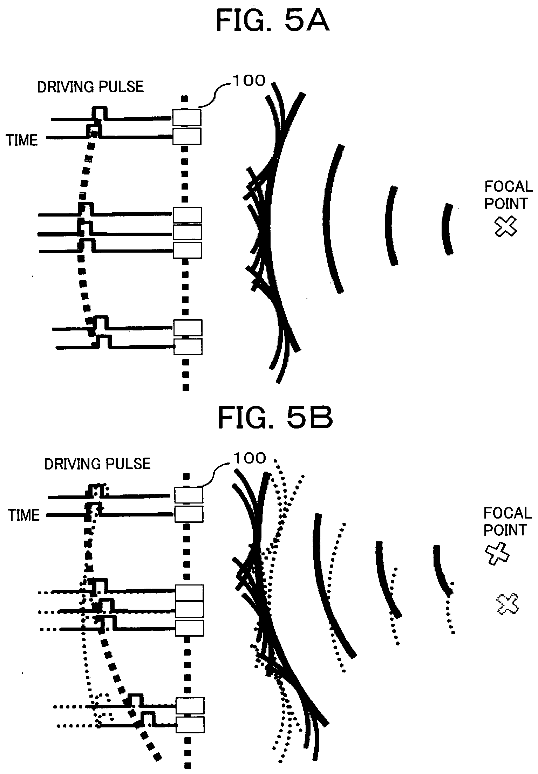

The displacement generating transmission beam forming unit 13 performs beam forming of the displacement generating ultrasonic wave. As FIGS. 5(a) and (b) show, the beam forming is conducted by obtaining the distance between the focal point and each element 100 of the ultrasound probe 1, and applying the delay time computed by dividing the difference in the distance between the elements by the sonic speed of the object for each element so as to transmit waves. FIGS. 5(a) and (b) clearly show that the position of the focal point F may be changed by controlling the delay time. When irradiating the focal point F with the displacement generating transmission beam, radiation pressure is generated in accordance with absorption and scattering of the ultrasonic waves in association with propagation. Generally, the radiation pressure is maximized at the focal point, and displacement is generated in the body tissues in the focal region. Upon interruption of radiation of the displacement generating transmission beam, the displacement amount will be moderated.

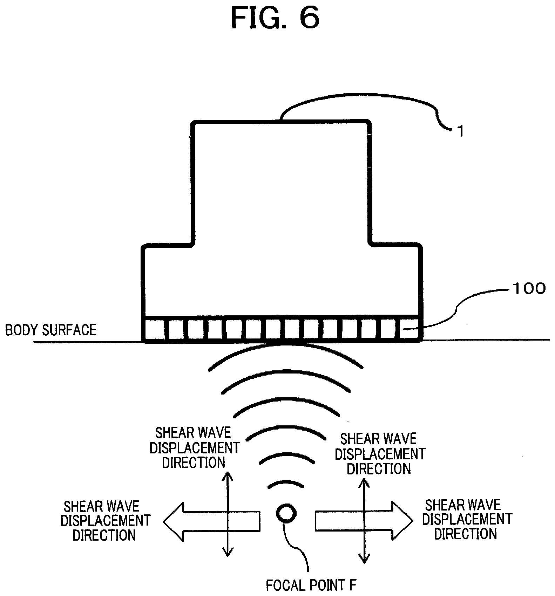

Referring to FIG. 6, the resultant radiation pressure generates the shear wave in the direction parallel to the test body surface, taking the focused point as a point of origin. As FIG. 6 shows, when the position of the focal point F is set, a raster used for detection of the shear wave propagation displacement (several μms to several tens μms) and a sampling point on the raster are determined. The PRF (Pulse Repetition Frequency) of received displacement detection beam for each raster is set to satisfy Nyquist Theorem with respect to the expected frequency of the shear wave. For example, if the raster is in the same direction as that of the shear wave displacement, the PRF is increased higher than the frequency of the shear wave twice or more.

When detecting the displacement of the shear wave in the region ROI_e, the second ultrasonic send-receive unit 30 adds the delay time for ultrasonic transmission and weight, and executes the process of converting the echo signal into the electric signal. The shear wave displacement computing unit 32 executes the displacement computing.

The aforementioned raster used for the displacement detection performs ultrasonic sending and receiving for the purpose of detecting the shear wave displacement. The process of ultrasonic sending and receiving is performed once before radiating the displacement generating transmission beam so as to obtain a reference signal for displacement calculation. Then the process of ultrasonic sending and receiving is performed a plurality of times for a period from a time point immediately after radiation of the displacement generating transmission beam to the time point at which the shear wave finishes propagation in the ROI_e so as to obtain a plurality of ultrasonic signals. The shear wave displacement computing unit 32 performs the correlation computing of a plurality of ultrasonic signals and the reference signal after radiation of the displacement generating transmission beam so that the displacement is calculated.

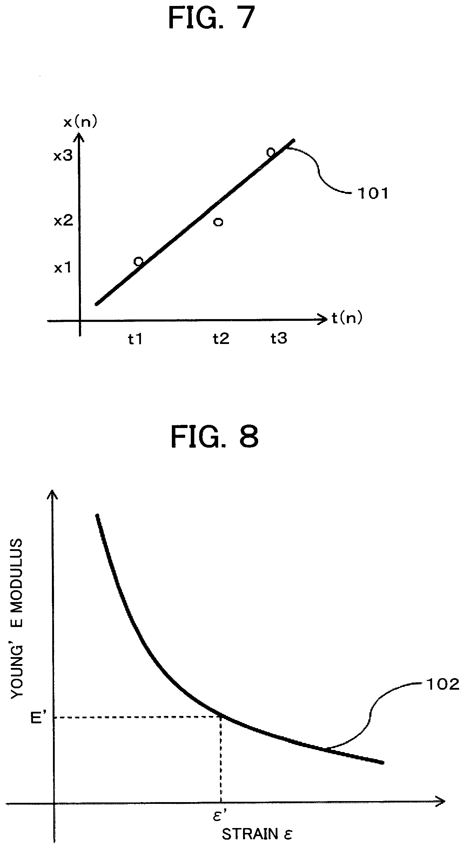

The elastic modulus computing unit 34 for computing the elastic modulus converts the displacement information calculated at a plurality of clock times into the temporal waveform information with respect to the shear wave displacement. The temporal waveform is obtained with respect to positions of a plurality of rasters x(n) (n=1,2,3,...) for observing the shear wave displacement along its propagation direction. At the time t(n), the shear wave displacement is maximized for each of the temporal waveforms at the respective positions x(n). A shear wave propagation speed c is estimated from the relational expression of x(n) and t(n). As FIG. 7 shows, the shear wave propagation speed is estimated from the gradient of a linear primary approximation line 101 formed by plotting the x(n) corresponding to t(n). The elastic modulus (Young's modulus) E is computed by substituting the shear wave propagation speed c for $E=3\rho c^2$, where $\rho$ denotes the density of the tissue subjected to the elastic modulus measurement.

In this example, the elastic modulus image is displayed in step S10 shown in FIG. 3. Generally, correlation among the elastic modulus E, strain $\varepsilon$ and stress $\sigma$ is expressed by the relational expression of $E=\sigma/\varepsilon$. If the stress in the strain distribution ROI_s is kept constant, the elastic modulus E and the strain $\varepsilon$ are brought into an inversely proportional relationship as a curve 102 in FIG. 8 shows. The stress is calculated from the average value $\varepsilon$' of the strain in the ROI_e where the elastic modulus is measured and the elastic modulus E' calculated in step S08. The elastic modulus distribution corresponding to the strain distribution is computed from the stress and the strain distribution derived from the generally employed method. The information about strain and the elastic modulus corresponding to the strain, or the stress information calculated using the relational expression of $E=\sigma/\varepsilon$ will be sent to the color scale setting unit 50 from the elastic modulus computing unit 34 via the central control unit 3. If the information of the strain, and the elastic modulus corresponding to the strain is input to the color scale setting unit 50, the color scale unit calculates the stress. If the stress calculated using the relational expression of $E=\sigma/\varepsilon$ is input to the color scale setting unit 50, the central control unit 3 computes the stress using the information of the strain and the elastic modulus corresponding to the strain, and the expression of $E=\sigma/\varepsilon$. The stress is calculated by the central processing unit functioning as the processing unit for executing the program.

The color scale setting unit 50 computes the elastic modulus distribution in the ROI_s corresponding to the strain distribution in the ROI_s as the first region (Region 1). The color scale setting unit 50 converts the strain color scale into the elastic modulus color scale.

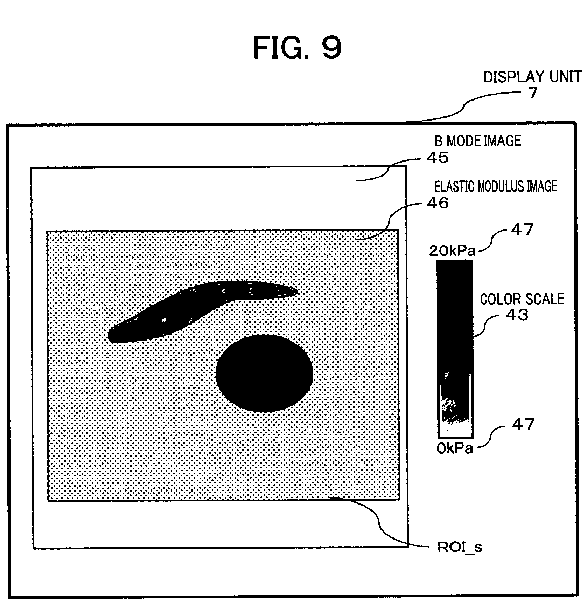

As FIG. 9 shows, the display unit 7 displays the B mode image 45, the elastic modulus image 46, and the color scale 43 indicating the elastic modulus information. The display range of the elastic modulus image 46 is the same as that of the previously shown strain image 41, and displayed in full color. Minimum and maximum values 47 of the elastic modulus (absolute value) expressed by the color scale 43 are displayed therearound. The method of determining the elastic modulus, and the method of calculating the minimum and maximum values of the color scale will be described in detail in reference to FIG. 8 when setting two effective regions of ROI_e for measuring the elastic modulus. Measurement results $\varepsilon 1$ and $\varepsilon 2$ of the strain distribution in the ROI1 and ROI2 are obtained. Then values of the elastic modulus E1 and E2 at the same position are obtained. Then the value $\sigma$ that satisfies the expression $E=\sigma/\varepsilon$ passing the two points is determined, resulting in the curve as shown in FIG. 8. The maximum and minimum values of the strain in the ROI_s, that is, $\varepsilon_{max}$ and $\varepsilon_{min}$ are obtained to provide $E_{max}$ and $E_{min}$ using the computed value of $\sigma$(based on the curve of FIG. 8). The values of the elastic modulus in the ROI_s may all be displayed in the color scale using the $E_{max}$ and $E_{min}$ as the maximum and minimum values of the color scale shown in FIG. 9. The explanation has been made with respect to two ROI_s. However, three or more ROI_s are applicable, or the least square fitting may be used to obtain the most probable value $\sigma$. It is assumed that the stress $\sigma$ is spatially uniform. However, it is possible to further improve the accuracy by monotonously decreasing the stress $\sigma$ in the depth direction if there are three regions ROI_e. In this case, the elastic modulus is expressed by the color scale. This method allows display of parameters concerning other dynamic elasticity besides the elastic modulus, for example, sonic speed of transverse wave and Poisson's ratio.

The hybrid method converts the strain information into the information of the absolute value of stiffness (elastic modulus and the like) over the entire area in the first region ROI_s where the image including the strain information as the relative value of stiffness is displayed. Then the image including the absolute value information of stiffness is displayed in the ROI_s. Upon elastic modulus measurement for displaying the elastic modulus image according to the example, the single point is set as the elastic modulus measurement point. Accordingly, it is possible to reduce the temperature rise of the body tissue around the focal point, and the ultrasound probe 1. It is further possible to form an image of the elastic modulus information in the region wider than the elastic modulus measurement range indicating the absolute value of stiffness. As the elastic modulus is measured by selecting the location with uniform stiffness, it is possible to measure the elastic modulus with high accuracy at the single location.

The value concerning the stiffness calculated in step S08 shown in FIG. 3, and the value concerning the stiffness displayed in step S10 may be regarded as amounts of absolute stiffness value, for example, shear wave propagation speed and shear elastic modulus ($=\rho c^2$) in addition to the elastic modulus.

It is judged whether or not the diagnosis using the hybrid method is finished in step S12. The signal indicating end of the hybrid method is input via a not shown input device. If the end signal has been input at the time when the determination is made with respect to the end of diagnosis, conduction of the hybrid method is finished in step S14. After the end of the hybrid method, the strain image derived from the general method is superposed on the B mode, and the resultant image is displayed, or only the B mode image is displayed.

If the diagnosis is not finished in step S12 of FIG. 3, the process returns to step S02 or S08. If the process returns to step S02 where the elastic modulus measurement position is selected, it is possible to select the point with uniform stiffness again. This may improve measurement accuracy and robustness against the body motion. Meanwhile, if the process returns to step S08, it is possible to reduce the time for signal processing and calculation cost. In this step, however, as the shear wave generating ultrasonic beam is radiated to the same location every time, safety cannot be ensured compared with step S02.

Referring to the processing flow shown in FIG. 3, the strain image and the elastic modulus image are alternately displayed with repetition. In step S08, if the elastic modulus is measured at the same location, the time interval of 1 to 2 seconds or longer is needed for the elastic modulus measurement for the purpose of suppressing the temperature rise in the body. This may result in the frame rate of the elastic modulus image of 1 or lower. Meanwhile, the frame rate of the strain image obtained in steps S02 and S04 is normally 10 approximately. It is preferable to update the strain image for the period from display of the strain image in step S04 to display of the elastic modulus image in step S10 for the purpose of keeping the real time ultrasonic image. For example, after updating the strain image 10 times, the elastic modulus image may be updated once. In this case, the elastic modulus measurement position selected in step S06 may be shifted in the image pickup profile while updating the strain image. In order to prevent such shifting of the elastic modulus measurement position, Motion Correction method (see H. Yoshikawa, et al., Japanese Journal of Applied Physics, Vol. 45, No. 5B, p. 4754, 2006) may be employed for correcting the elastic modulus measurement position whenever necessary.

The elastic modulus measurement position may be selected in step S06 of the processing flow shown in FIG. 3 by displaying the standard deviation S of the strain amount as a result of the signal processing, and distribution of the difference between maximum and minimum values of the strain amount, that is, Max-min on the display unit 7 in monochrome or in full color, and allowing the operator to determine the elastic modulus measurement position using the not shown input device while observing the displayed image. When the operator manually inputs, the elastic modulus measurement position may be determined while avoiding such part as the blood vessel. Alternatively, the elastic modulus measurement position may be selected by the operator using the not shown input device while observing the strain image displayed in step S04 without executing the signal processing. The determined position information is input to the central control unit 3. Upon determination of the measurement position, the operator is allowed to control the strain amount, standard deviation, and gradation of the Max-min in monochrome or full color into the range from 3 to 256 so as to be displayed on the display unit 7. The gradation is changed using the not shown input device. Setting the gradation to 3 allows easy selection of the position with uniform strain amount. Setting the gradation to 256 allows accurate selection of the measurement point. If the operator determines the elastic modulus measurement position manually, it is possible to omit the measurement position selection unit 40. The kernel K or the filter G may be formed into a circular shape, elliptical shape, square shape, and any other geometric shape in addition to the rectangular shape.

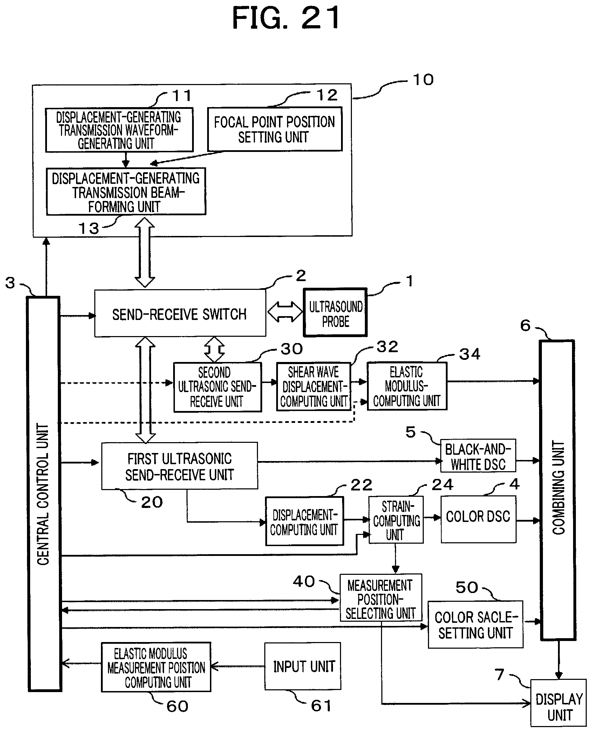

The explanation has been made with respect to the configuration that processing results executed by the measurement position selecting unit 40 of the ultrasound diagnostic system according to the example are sent to the central control unit 3. A system configuration shown in FIG. 21 may be practical as a modification of the present example. The modified system is made by newly adding an elastic modulus measurement position computing unit 60 and an input unit 61. According to the modified example, the processing results of the measurement position selecting unit 40 is displayed on the display unit 7, and the operator inputs the selection result of the elastic modulus measurement position to the elastic modulus measurement position computing unit 60 via the input unit 61 in reference to the displayed image.

According to the modified example, the measurement position selecting unit 40 is configured to display all the part where the strain uniformity in the ROI exceeds a predetermined threshold value as a recommended part of the elastic modulus measurement position rather than displaying the location with maximum strain uniformity in the ROI as the optimal part. The elastic modulus measurement position is determined in accordance with the information input by the operator via the input unit 61 from the range of the recommended part. The threshold value may be set by multiplying the maximum value of the strain uniformity in the ROI by such coefficient as 0.8. The method based on statistical information may be used, which calculates the uniformity for each location in the image to determine the threshold value from the histogram. The configuration which allows the operator to make a final selection ensures that the criterion other than the uniformity is added for selection of the elastic modulus measurement position. This makes it possible to avoid selection of the position near the part suspected as the diseased site, or the part such as bone, which is required to be kept from exposure to strong ultrasonic waves.

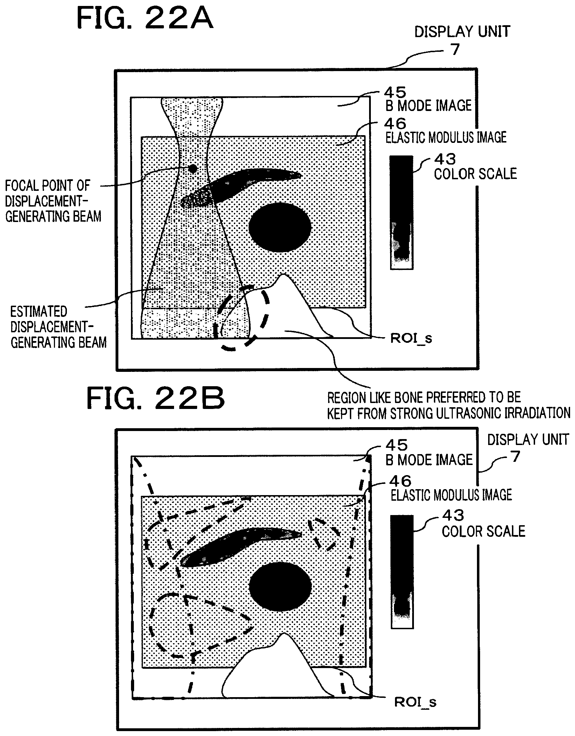

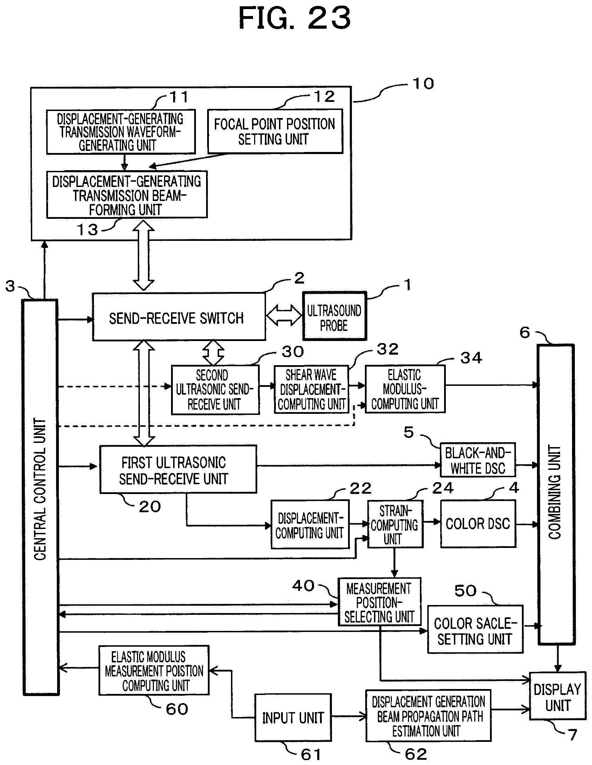

Another modified example will be described referring to FIGS. 22 and 23. This example presents the method of accurately determining the part preferred to be kept from being exposed to radiation of the displacement generating ultrasonic wave, for example, bone and blood vessel. Assuming that the position shown in FIG. 22A is set as the focal point of the displacement generating beam, it is possible to calculate a shape of the displacement generating transmission beam from the opening aperture and frequency. The position exposed to the strong ultrasonic wave is estimated by adding the value derived from estimating the organic attenuation factor to the calculated result.

Referring to the system configuration shown in FIG. 23, the computation is performed by a displacement generating beam propagation path estimation unit 62 further added to the system configuration shown in FIG. 21. A comparison is made between the position of bone, for example, that is preferred to be kept from strong radiation, which has been input by the operator from the input unit 61 and the position exposed to the strong ultrasonic wave on the image. If overlapping between the part to be kept from radiation and the displacement generating beam exceeds a predetermined threshold value, it is judged that the focal point position of the displacement generating beam is undesirable. Sequential execution of the aforementioned process makes it possible to distinguish the suitable part for the elastic modulus measurement from the unsuitable part.

The result may be displayed on the image as shown by the region enclosed by a dashed line of FIG. 22B. The region enclosed by the dashed line indicates the recommended elastic modulus measurement position estimated from the part to be kept from radiation. Meanwhile, the recommended elastic modulus measurement position determined from the strain uniformity is enclosed by a dotted line as shown in FIG. 22B. The aforementioned two kinds of recommended elastic modulus measurement positions allow the apparatus to determine the final elastic modulus measurement position or the operator to judge about the elastic modulus measurement position. The displacement generating transmission beam shape may be estimated using a general attenuation or the value estimated by the first ultrasonic send-receive unit 20 from the echo signal.

The explanation has been made that the object to be measured is the inside of the test body. However, it is effective to use the method which interposes a coupler (polymer gel) as a material with known elastic modulus between the ultrasound probe and the test body to set so that the ROI_s includes the coupler and at least one of the regions ROI_e is set in the coupler. By including the measurement point with the known elastic modulus, it is possible to significantly improve the measurement accuracy.

As for the uniformity which has not been explained in detail so far, the strain with 1 to 10% of the strain difference between maximum and minimum values in the ROI_s may be appropriate to be considered as substantially uniform. If the number of the regions ROI_e is small, it is preferable to have dispersion value indicating the uniformity as small as possible. Meanwhile, if the number of the regions ROI_e is large, the accuracy may be ensured by the least square fitting. It is therefore possible to effectively perform the measurement even if the dispersion value is rather large. It is to be noted that this and other examples include substantially uniform states.

Second Example

A second example presents an ultrasound diagnostic apparatus using an ultrasound probe that sends the ultrasonic beam to the test body and receives the echo signal. The apparatus includes a strain computing unit 24 which radiates a first displacement detection beam, and computes strain information in a region 1 based on the echo signal from the test body that receives the first displacement detection beam, a displacement generating unit which radiates a focused beam to an inside of the test body to displace a tissue in the test body, an elastic modulus computing unit 34 which radiates a second displacement detection beam, and detects a shear wave displacement generated by the focused beam based on the echo signal from the test body that receives the second displacement detection beam for detecting an elastic modulus in a region 2 included in the region 1, and a display unit 7 which displays a strain image based on the strain information and the elastic modulus, and the measurement position selecting unit 40 that selects at least one elastic modulus detection position at which the elastic modulus is detected based on the strain information. The measurement position selecting unit 40 selects the single elastic modulus detection position with uniform strain distribution so that two different focal point positions to which the focused beam is radiated are determined from the selected elastic modulus detection position.

The displacement generating unit of the aforementioned ultrasound diagnostic apparatus according to the example includes a beam time setting unit 14 that sets the focused beam transmission time. The beam time setting unit 14 sets the transmission time to achieve the same cycle for ON/OFF switching of the focused beam radiated to the two different focal point positions adjusting the ON/OFF switching cycle to generate the frequency or phase chirp signal.

The ultrasound diagnostic apparatus and the ultrasound diagnostic system for measuring the elastic modulus using the burst-chirp method will be described as the second example.

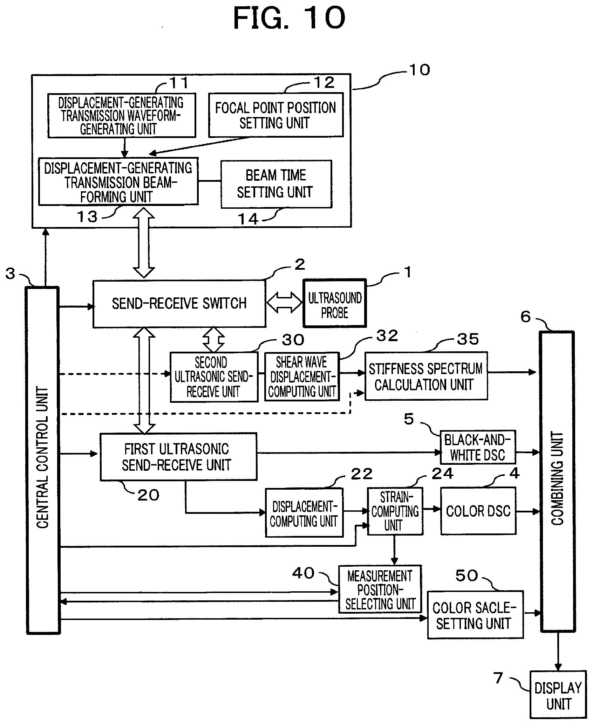

Fig. 10 shows a specific example of an overall structure of the ultrasound diagnostic system for implementing the second example. The system structure is different from that of the first example shown in FIG. 1 in the beam time setting unit 14 added to the displacement generating unit 10, and a stiffness spectrum calculation unit 35 provided in place of the elastic modulus computing unit. An output of the shear wave displacement computing unit 32 is input to the stiffness spectrum calculation unit 35 for calculating the value that relates to the stiffness. The stiffness spectrum calculation unit 35 may be realized by executing the program of the Central Processing Unit (CPU) functioning as the processing unit.

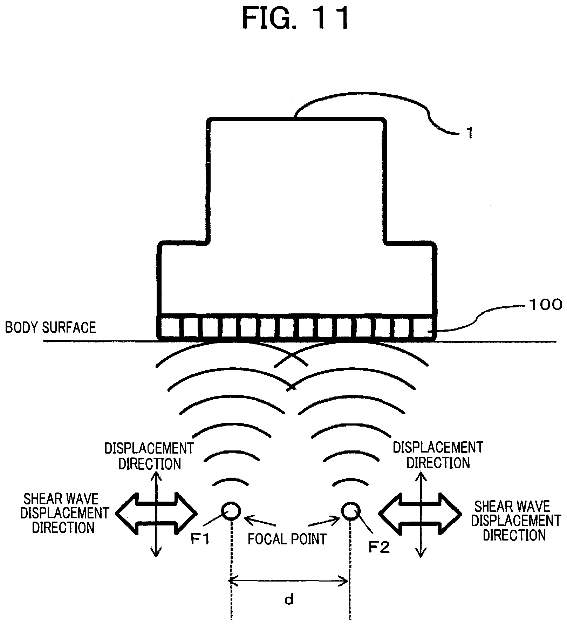

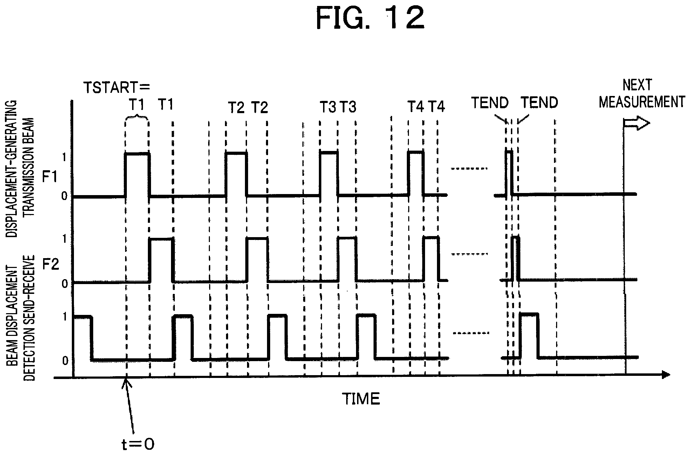

Transmission of the displacement generating focused beam using the burst-chirp method will be described referring to FIGS. 11 and 12. Two displacement generating transmission beams are controlled so as to generate the displacement at focal points F1 and F2 of the test body tissue alternately. The ON/OFF switching of radiation of the displacement generating transmission beam to the respective focal points is controlled by the central control unit 3. The newly added beam time setting unit 14 sets the ON/OFF switching time.

FIG. 12 shows sequences of the displacement generating transmission beam and the displacement detection send-receive beam. The sequence of the displacement generating transmission beam shown in the drawing is obtained when sweeping the value of a switching cycle Tm from a large value to a small value, that is, the interval $\Delta Tm(=T(m+1)-Tm)$ between the switching cycles Tm and T(m+1) is a negative constant. The displacement generating ultrasonic beam transmission method herein will be referred to as the burst-chirp method. Meanwhile, the central control unit 3 controls the second ultrasonic send-receive unit 30 so as to perform ON/OFF switching of radiating the displacement detection send-receive beam.

It is assumed that the time when the first displacement generating transmission beam is radiated is set to zero, that is, t=0. First, the displacement detection send-receive beam is switched to ON(=1), and a reference signal is obtained. The reference signal is used for computing the shear wave displacement by the shear wave displacement computing unit 32. In the state where the displacement generating transmission beam to the focal point F2 is OFF (=0), the displacement generating transmission beam to the focal point F1 is set to ON (=1) so that displacement is generated at the focal point F1, and the shear wave propagates. The displacement generating transmission beam to the focal point F1 is constantly kept ON when $0 \leq t \leq T1$. At the time of t=T1, the displacement generating transmission beam at the focal point F1 is switched to OFF. At this time, the displacement generating transmission beam at the focal point F2 is switched to ON. Displacement is generated at the focal point F2, and the shear wave propagates. The displacement generating transmission beam to the focal point F1 is in OFF state, and the displacement generating transmission beam to the focal point F2 is in ON state at the time of $T1 \leq t \leq T1+T1$. In the aforementioned sequence, the switching cycle between the two displacement generating transmission beams is T1. At the end of radiation of the displacement generating transmission beam to the two focal points, the displacement detection send-receive beam is switched to ON (=1).

Radiation of the displacement generating transmission beam to the two focal points and sending and receiving of the displacement detection send-receive beam are repeated by changing the ON/OFF switching cycle Tm for the displacement generating transmission beam. In this case, "m" (=1, 2, 3...) denotes the cycle at which the focal points F1 and F2 are brought into ON at the mth time. The acoustic intensity of the burst signal to the respective focal points may be at the same level or the different level. In association with radiation of the displacement generating transmission beam, the shear waves generated at the focal points F1 and F2 interfere with each other while propagating, and further cancel or amplify with each other. Meanwhile, heat is generated at the respective focal points simultaneously with the displacement.

Referring to FIG. 11, the distance between the two focal points is set to d. As the value of d becomes small, that is, the distance between the focal points F1 and F2 is reduced, the degree of interference is increased. As the focal distance is decreased, a temperature rise E between the focal points becomes larger than the temperature at the focal point owing to thermal conductivity, thus deteriorating safety. On the contrary, as the d is increased, the temperature rise is suppressed to improve safety. However, the degree of interference is reduced. Accordingly, the optimal value for the d is obtained when the maximum value of the temperature rise is the same as the maximum value of the temperature rise at each focal point, and the wave interference occurs. The optimal value of d is dependent on the depth of the focal point, time for radiating the displacement generating transmission beam, frequency, and diagnostic part (influences sonic speed of the body, ultrasonic absorption, and heat conductivity). As the thermal conductivity of the body is approximately 0.6 W/m/K, the range of the temperature rise around the focal point is at the level similar to the width of the displacement generating transmission beam when the radiation time is several milliseconds. The distance d, thus needs to be equal to or larger than the width of the displacement generating transmission beam. In this case, the displacement generating transmission beam has the width that is substantially the same as the beam width of the focusing type transducer, which is set as the diameter of the region (circle) where the energy density of the ultrasonic wave at the focal point position becomes zero for the first time.

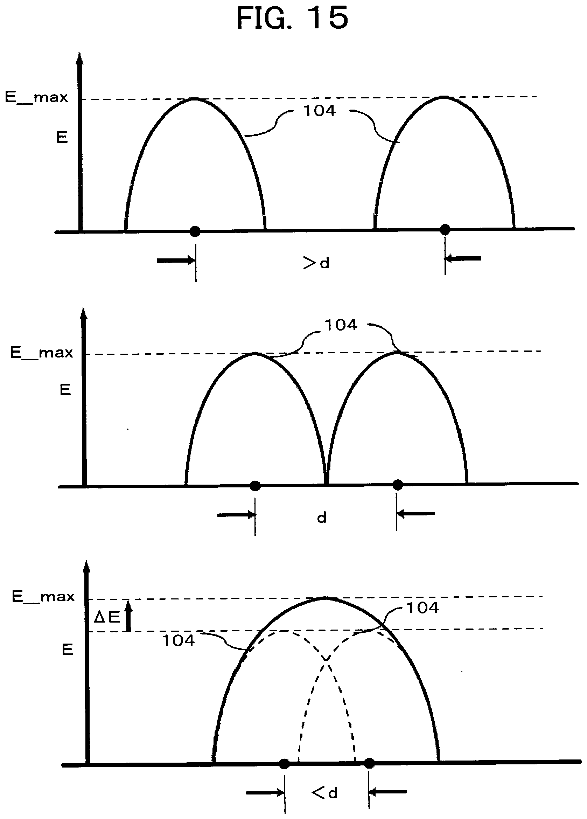

FIG. 15 shows a relationship between the distance d and the temperature rise E. As a wave profile 104 indicating the temperature rise at the respective focal points shows, the temperature rise at each focal point is maximized at the focal point position. Then at the location apart from the beam width d, the temperature rise becomes zero. As the intermediate and the upper graphs show, when the distance d is equal to or larger than the beam width, the maximum value of the temperature rise is the same as the maximum value of E_max of the overall temperature rise obtained by summing up the respective temperature rises. As the lower graph shows in the drawing, when the distance d is smaller than the beam width, the maximum value E_max of the overall temperature rise is increased to be larger than the maximum value of the temperature rise at each focal point indicated by the dotted lines by $\Delta E$. It is clearly understood that making the distance d equal to or larger than the beam width keeps safety concerning the temperature rise.

Setting of the optimal value d upon measurement will be described hereinafter. When diagnosing the liver, the beam layer is 1.8 mm when the focal point depth is 4 cm, the F value is 1, and the carrier frequency is 2 MHz. The F value is calculated as the focal point depth/opening aperture, and the beam width is calculated as (2.44 * F value * wavelength of the carrier signal). When the beam radiation time, that is, average value of the switching cycle is 180 $\mu$s, and the shear wave propagation speed is 1 m/s, the wavelength $\lambda$ of the shear wave is approximately 0.2 mm. Furthermore, at the carrier frequency, the maximum value of the shear wave propagation distance that can be detected based on experimental data is approximately 6 mm. Considering those values, the value of d is set so as to establish the relationship of 10□ < d < 30□ using the beam width, the maximum propagation distance and the shear wave wavelength. Likewise, in diagnosing the breast, if the focal point depth is 2 cm, the F value is 1, and the carrier frequency is 7 MHz, the result of calculating the beam width is 0.5 mm. If the average value of the switching cycle is 110 $\mu$s, and the shear wave propagation speed is 1 m/s, the shear wave wavelength $\lambda$ is approximately 0.1 mm. At the carrier frequency, the maximum value of the shear wave propagation distance that can be detected based on the experimental data is approximately 3 mm. Accordingly, the value d is set so as to establish the relationship of 5$\lambda$ < d < 30$\lambda$. The central control unit 3 reads the value d from the not shown memory, and sets the value in the focal point position setting unit 12. The value concerning the switching cycle is determined from the value propagation speed predicted as the set value of d.

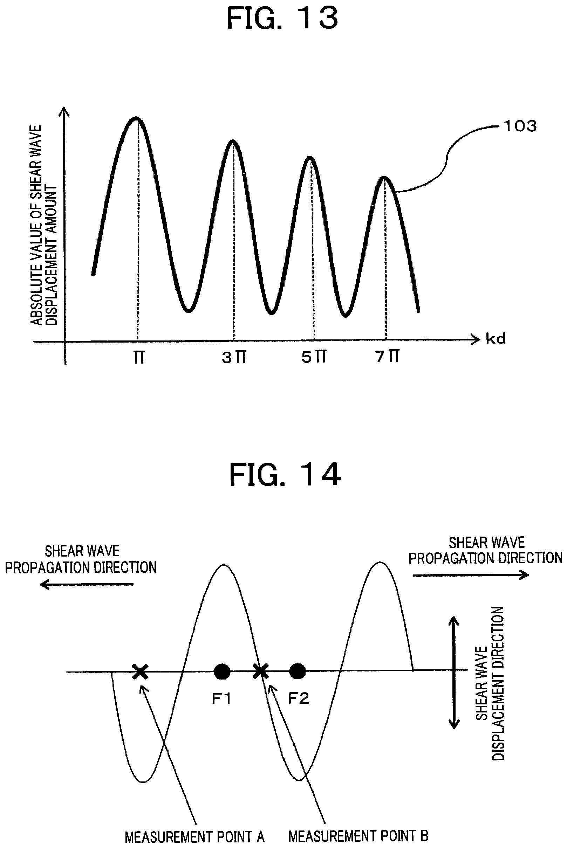

As described above, in the second example, the switching cycle when the amplitude is increased by interference between the shear waves generated at the two focal points is obtained while changing the switching cycle so as to measure the elastic modulus. The condition for amplifying the interference wave will be described. An expression of fm = 1/Tm is set where the fm denotes the switching frequency (repetitive frequency) as a reciprocal of the switching cycle Tm. The condition where the interference wave is amplified to allow the absolute value of the displacement amount to reach a peak value (maximum value) is established when the distance d between the two focal points is (n+1/2) times longer than the wavelength $\lambda$. It may be expressed by a curve 103 shown in FIG. 13, and expression 1. The switching frequency fm in this case is expressed as fM(n).

$$k*d = (2\pi fM(n)/c)*d = 2\pi(n+1/2) \; … \; \text{expression 1}$$

where k denotes the number of waves (=$2\pi/\lambda$), c denotes the shear wave propagation speed, and n denotes zero or a positive integer (n=0, 1, 2, ...). The shear wave propagation speed has the value unique to the tissue property.

Assuming that the value of the switching cycle Tm as the peak value is set to TM(n), the expression of TM(n) = 1/fM(n) is established. Then an expression 2 is derived from the expression 1.

$$TM(n) = d/c*(2/(2n+1)) \; … \; \text{expression 2}$$

For example, in the state where n=1 and d=2 [mm], if c=1 [m/s], the relationship of TM(1)=1.3[ms] (fM(1)=750[Hz]) is established. If c=5 [m/s], the relationship of TM(1) = 0.3 [ms] (fM(1)=3.8 [kHz]) is established. As described above, the shear wave propagation speed c is dependent on the tissue stiffness. As the stiffness degree is higher, the value c is increased. It is therefore possible to estimate the shear wave propagation speed and the tissue stiffness such as the elastic modulus from the value of the TM(n). It is preferable to control the ON/OFF switching cycle Tm of the displacement generating transmission beam to be in the range from several tens Hz to several kHz. The stiffness is estimated based on the TM(n) with larger n so that the total radiation time of the displacement generating focused beam is reduced, thus suppressing the temperature rise. It is preferable to allow the central control unit 3 to control the ON/OFF switching of the displacement generating transmission beam to the respective focal points so that the total radiation time of the displacement generating transmission beam to the respective focal points is equal to or shorter than 1 ms.

The feature of the technology is that the elastic modulus is measured by controlling the time for ON/OFF rather than the carrier signal cycle of the displacement generating transmission beam. Accordingly, the carrier frequency is increased to ensure image pickup with narrow beam width and high spatial resolution.

The stiffness spectrum calculation unit 35 performs a spectrum analysis with respect to the output signal from the shear wave displacement computing unit 32, obtains the fM by which the amplitude value is maximized and the corresponding TM, and further calculates the value concerning the stiffness such as the shear wave propagation speed, the elastic modulus, and the shear elastic modulus.

If the acoustic intensity with respect to each focal point is the same, the case where interference occurs between the shear waves generated at the two focal points may generate larger displacement compared with the case where the displacement generating transmission beam is radiated only to the single focal point.

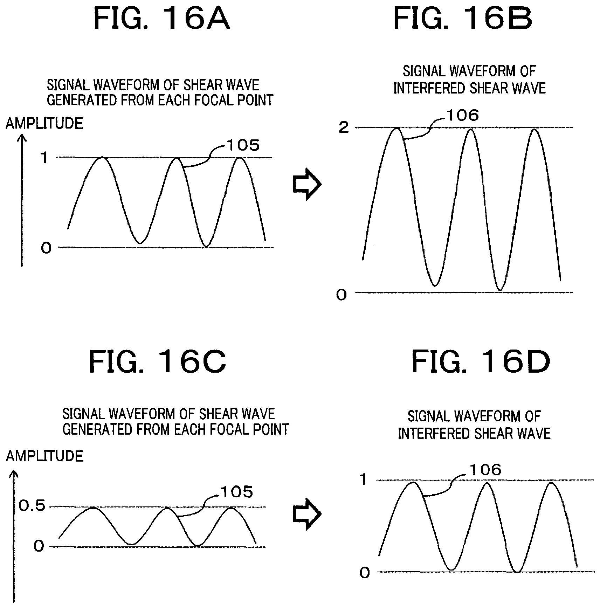

FIGS. 16A-16D show signal waveforms of the shear wave generated at the single focal point, and an interference waveform of the shear waves generated at the two focal points. It is assumed that the minimum amplitude derived from observation of the shear wave displacement resulting from radiation of the displacement generating transmission beam to the single focal point is set to 1. Referring to FIG. 16A, when the shear wave amplitude generated by the displacement generating transmission beam to each focal point is 1, the amplitude of the interference wave is larger than the one before interference (ideally, larger by twice) when the switching cycle reaches the TM as shown in FIG. 16B. The displacement generating efficiency with respect to transmission, that is, the transmission sensitivity is enhanced. When safety is required to be emphasized, the amplitude is set to 0.5 so as to decrease the acoustic intensity of the displacement generating transmission beam to each focal point as FIGS. 16(c) and (d) show. When the shear wave amplitude before interference is smaller than 1, the shear wave displacement cannot be measured. If the shear wave amplitude before interference is larger than 0.5, the amplitude of the interference wave at the switching cycle of TM is increased (ideally larger by twice), and the amplitude is equal to or larger than 1. This makes it possible to detect the displacement.

If the frequency and the shape of the displacement generating transmission beam are the same, the method of improving safety is available by setting the time for bringing the displacement generating transmission beam into OFF state by the last n % (n=positive real number) of the time Tm for which the displacement generating transmission beam is switched to ON state besides the method of reducing the acoustic intensity by controlling the amplitude of the displacement generating transmission beam for generating the displacement. In this case, it is to be noted that the time set for bringing OFF state will not change the switching cycle Tm.

As FIG. 14 shows, it is necessary to set the displacement detection point to the location such as an observation point A so as not to set such point to the location where the displacement is minimized like an observation point B. When observing a transitional phenomenon of switching radiation of displacement generating transmission beam to ON state only once, the aforementioned case is not so important. However, when using the interference between the shear waves by taking the two focal points as the audio sources, the maximum and minimum points of the absolute values of the displacement amount(=amplitude value) distribute alternately. The location at which the absolute value of the displacement amount is assumed to be maximized is selected for the raster which monitors the displacement, or a plurality of monitoring points are set so that the maximum point is included in the observing point. When monitoring at a plurality of points, the absolute value of the difference between the displacement at the maximum point and the displacement at the minimum point may be set as the displacement amount.

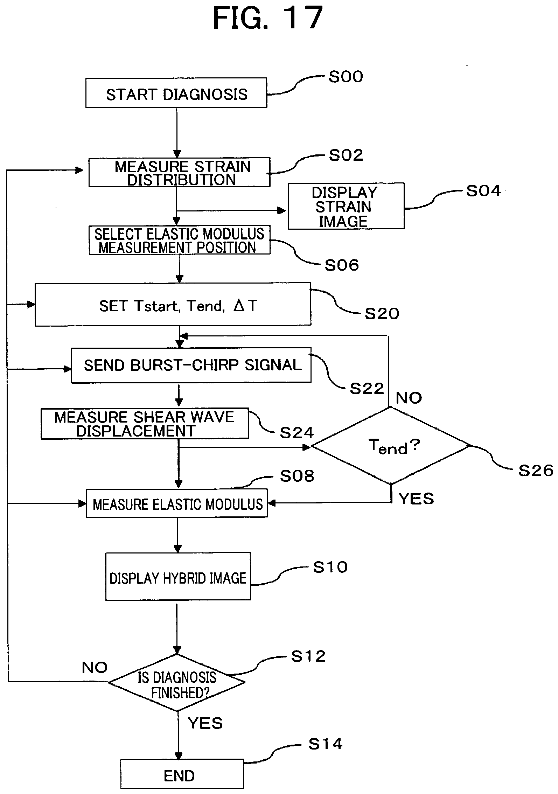

A processing flow of the hybrid method according to the second example will be described referring to FIG. 17. The process executed in steps S00 to S04, and steps S10 to S14 are the same as those of the processing flow according to the first example shown in FIG. 3, and explanations thereof, thus will be omitted. Steps S06 and S08 different from those of the first example will only be explained.

In step S06, positions of the focal points F1 and F2 are set. Upon setting of the two focal points, the center between the two focal points is set as POI (Point of Interest)(the center of the line that connects a pair of two focal points), and the distance therebetween is set. The distance between the two focal points is set to the value shorter than the distance where two shear waves interfere with each other, and larger than the width of the displacement generating transmission beam radiated to the respective focal points.

If the optimal elastic modulus measurement position is set to P(x1, y1) as shown in FIG. 4A, the coordinate of the POI is determined as POI(x1, y1), and the distance is determined as lx. Information of the POI and the distance is set by the focal point position setting unit 12. In this case, coordinates of the two focal points are set to $F(x1-\Delta x, y1)$ and $F(x1+\Delta x, y1)$, respectively, where $\Delta x=lx/2$.

If the image processing is performed using the filter G to select the optimal region R(n), and a position of center of gravity P of the R(n) is selected as the optimal elastic modulus measurement position, the coordinate of the POI is determined as POI(x2, y2), and the distance is determined as lx. The information of POI and distance is set by the focal point position setting unit 12.

When the distance is set, the value of n in the expressions 1 and 2, and the optimal observing point are determined in accordance with the estimated shear wave speed. The observing point is defined based on the maximum point of the absolute value of the shear wave displacement, or a plurality of positions including the maximum point in the shear wave propagation distance. The observing point is read from the not shown storage medium, and then set. The raster for detecting the amplitude (several nm to several tens nm) of the shear wave propagation on the observing point, and sampling points on the raster are determined. The received PRF (Pulse Repetition Frequency: frequency of repeatedly transmitted pulse) of the displacement detection beam for each raster is set to satisfy Nyquist Theorem with respect to the expected shear wave frequency. If the raster is in the same direction of the shear wave displacement, the PRF is set to be twice the shear wave frequency. The determined n and the observing position may be displayed on the screen.

In step S20, an initial value $T_{start}$, a final value $T_{end}$ and an interval $\Delta T$ of the switching cycle of the displacement generating transmission beam radiated to the two focal points are set. The initial value $T_{start}$ and the final value $T_{end}$ are set to values which lead to the peak in the range where the expressions 1 and 2 are satisfied with respect to the measurement point and the distance d between the two focal points. The initial value $T_{start}$, the final value $T_{end}$ and the interval $\Delta T$ are read from the not shown storage medium by the central control unit 3 in accordance with the measurement point, depth and the distance between the focal points so as to be set in the beam time setting unit 14.

Then in step S22, the reference signal used for correlation operation upon the shear wave displacement detection is obtained on the raster for detecting the amplitude of the shear wave propagation. Then the burst-chirp signal is sent at the switching cycle of $T1=T_{start}$ so as to generate the sear waves at the two focal points.

In step S24, the ultrasonic signal for detecting the shear wave is received on the raster for detecting the amplitude of the shear wave propagation. Each displacement at the respective measurement points may be constantly detected during the period from the timing at which the burst-chirp signal is switched to OFF to the timing at which the shear wave reaches and passes over every observing point. Alternatively, the period taken for the shear wave to reach and pass over the observing point is preliminarily obtained from the distance between the focal point and the observing point, and the estimated shear wave speed so that the displacement is detected during such period. The latter case may make the PRF high, thus allowing the highly accurate displacement detection. The second ultrasonic send-receive unit 30 extracts the received signal corresponding to the fm through the signal processing such as the band pass filter, and then the shear wave displacement computing unit 32 performs the known correlation operation to calculate the shear wave displacement. The correlation operation denotes the calculation performed using the reference signal and the echo signal received through the displacement detection beam for each time. This calculation provides the temporal waveform of the shear wave amplitude at each of the observing points.

In step S26, it is judged whether the previous switching cycle Tm is $T_{end}$. If it is not $T_{end}$, the process returns to step S22 where the burst-chirp signal is sent at the next Tm+1 switching cycle. In step S22, when obtaining the reference signal again, robustness of the correlation operation is high resulting from shifting of the focal point position during the measurement. Upon transmission of the burst-chirp signal subsequent to the Tm+1 switching cycle, the measurement time may be reduced by omitting step of acquiring the reference signal, and performing the correlation operation using the reference signal that has been first obtained.

If it is judged that the Tm is $T_{end}$, the process proceeds to step S08 shown in FIG. 17 where the stiffness spectrum calculation unit 35 calculates the value concerning the stiffness such as the shear wave propagation speed, the elastic modulus, and the shear elastic modulus. If the diagnosis is not finished in step S12 shown in FIG. 17, the process returns to step S02, S08, S20 or S22.

A variant type of the elastic modulus measurement using the burst-chirp method will be described hereinafter.

Referring to FIG. 13, if a plurality of peaks can be observed in the elastic modulus measurement of a pair of two focal points, it is possible to estimate the elastic modulus from the average value of a plurality of values of c obtained using the expression 2 in reference to a plurality of the switching cycles TM(n). It is also possible to estimate the elastic modulus from the interval ΔTM=TM(n+1)-TM(n) of the switching cycles TM(n) as the peaks. Alternatively, the elastic modulus may be estimated from the average value of the intervals ΔTM of a plurality of the switching cycles. The interval ΔTM is obtained by an expression 3 derived from the expression 2 as follows.

$$\Delta TM = d/c * (-4/((2n+1)*(2n+3))) \ \ldots \ \text{expression 3}$$

Switching of the displacement generating transmission beam to the two focal points is controlled to ON/OFF alternately. It is also possible to bring the state into ON/OFF simultaneously so as to generate the displacement at the same time. In this case, the condition that the interference wave is amplified to have the peak value is established when the distance d is (n+1) times longer than the wavelength A. Therefore, the following expression 4 is established corresponding to the expression 1.

$$k*d = (2\pi f/c)*d = 2\pi(n+1) \ \ldots \ \text{expression 4}$$

The elastic modulus measurement method with high accuracy may be employed as described below. That is, the TM is obtained through measurement by roughly setting the interval ΔT of the switching cycle in the first measurement. Then the interval □T is set at the frequency around the TM in more detail to obtain the more accurate value of the TM in the next measurement.

The switching cycle Tm may be changed to the next switching cycle T(m+1) after repeating several ON/OFF switching operations at the same Tm rather than executing the single ON/OFF control. Repeating of several ON/OFF switching operations makes it possible to realize the measurement with further high sensitivity.

In this case, the Tm value has been changed from the large value to the small value. It may be changed conversely from the small value to the large value. The interval ΔTm may be changed based on an arbitrary function such as a geometric series rather than the fixed value.

In this case, the method of estimating the elastic modulus from the peak value (maximum value) has been described. However, the minimum value may be used for such estimation. In such a case, values derived from expressions of kd = 2n, 3π,... may be used as shown in FIG. 13. It is also possible to estimate the elastic modulus from the interval between the maximum and minimum values. The interval between the maximum and the minimum values becomes half the interval ΔTM of the peak value as indicated by the expression 3. This makes it possible to reduce more time compared with the measurement of the interval between the peak values.

The method of radiating the displacement generating transmission beam to the two focal points has been described. It is possible to set two or more focal points (for example, four) at equal intervals on the single line in the body so as to radiate the displacement generating transmission beam to every other one of the focal points in the same sequence as the focal point F1 or F2 (for example, performing the sequences of the focal points F1, F2, F1, and F2 to four focal points sequentially from the end). As a result, the number of the interfering waves is increased, thus improving the sensitivity while keeping safety.

The measurement method by fixing the switching frequency and changing the distance d between the focal points may be considered to be available. This method allows change in the focused position of the displacement generating transmission beam, thus ensuring further safe measurement.

Use of the random wave including a plurality of the switching frequencies fm of the burst instead of beam transmission by selecting the fm allows execution of radiation of the displacement generating transmission beam and send-receive of the displacement detection beam only once. A spectrum analysis is performed after calculation of the shear wave displacement to calculate the displacement with respect to each of a plurality of the fms. This makes it possible to reduce the measurement time.

In the case where three or more focal points are set, the burst-chirp method may be applied to allow the shear wave displacement to be increased using interference of the wave while keeping the acoustic intensity at each focal point. This makes it possible to improve safety and sensitivity of the shear wave displacement detection. The larger the number of the focal points becomes, the more the effect of the burst-chirp method is improved. Third Example

The hybrid method according to a third example derived from combining the first and the second examples will be described. The not shown system configuration of the third example is formed by adding the beam time setting unit 14 and the stiffness spectrum calculation unit 35 to the system configuration shown in FIG. 1. The system is configured to input an output signal from the shear wave displacement computing unit 32 to both the elastic modulus computing unit 34 and the stiffness spectrum calculation unit 35 which are arranged in parallel. The central control unit 3 is connected to both the beam time setting unit 14 and the stiffness spectrum calculation unit 35. The operator selects any one of the elastic modulus computing unit 34 and the stiffness spectrum calculation unit 35 arranged in parallel, and uses the selected one. The elastic modulus computing unit 34 and the stiffness spectrum calculation unit 35 will be generically referred to as an elastic modulus computing unit in the specification.

According to the third example, the elastic modulus is measured using any one of the method described in the first example (relationship between the shear wave observing position $x(n)$ and the time $t(n)$ at which the shear wave displacement is maximized) and the burst-chirp method described in the second example in step S08 of the processing flow of hybrid method shown in FIG. 17. The operator determines as to which method is employed for the elastic modulus measurement using the not shown input device, for example. Alternatively, another method is available for automatic determination by the depth of the elastic modulus measurement position selected in step S06, carrier frequency of the shear wave generating ultrasonic beam, F value of the shear wave generating ultrasonic beam (= focal distance/opening aperture), time for radiating the shear wave generating ultrasonic beam, and measurement point (mammary gland, liver, prostate gland, blood vessel). If the determination is made automatically, the central control unit 3 reads the optimal elastic modulus measurement method adapted to the depth of the elastic modulus measurement position, the carrier frequency of the shear wave generating ultrasonic beam, and the measurement point. Combining the first and the second examples allows application of the burst-chirp method when the point to be measured requires more safe measurement (near the blood vessel), or when the number of channels available for the ultrasound probe 1 is small, and the acoustic intensity value of the radiating focused ultrasonic beam is smaller than the threshold value.

The burst-chirp method will be applied in various cases where the time for radiating the shear wave generating ultrasonic beam is smaller than the threshold value, the F value of the shear wave generating ultrasonic beam is larger than the threshold value, the depth of the elastic modulus measurement position from the body surface is larger than a certain value, or the value expressed by the function of those values and the carrier frequency of the shear wave generating ultrasonic beam is larger than the threshold value.

Fourth Example

A fourth example provides the ultrasound diagnostic apparatus which uses the ultrasound probe for sending the ultrasonic beam to the test body and receiving the echo signal. The apparatus includes a strain computing unit which radiates a first displacement detection beam, and computes strain information in a region 1 based on the echo signal from the test body that receives the first displacement detection beam, a displacement generating unit 10 which displaces a tissue inside the test body by radiating a focused beam to the inside of the test body, an elastic modulus computing unit 34 which radiates a second displacement detection beam, and detects a shear wave displacement generated by the focused beam based on the received echo signal from the test body so as to detect the elastic modulus in the region 2 included in the region 1, the display unit 7 that displays a strain image based on the strain information and the elastic modulus, and a measurement position selecting unit 40 that selects at least one elastic modulus detection position at which the elastic modulus is detected based on the strain information. The measurement position selecting unit 40 selects a plurality of elastic modulus detection positions at which the strain is uniformly distributed. At least one focal point position irradiated by the focused beam is determined from a plurality of elastic modulus detection positions selected by the measurement position selecting unit 40.

Each system according to the first to the third examples has only one optimal elastic modulus measurement position output from the measurement position selecting unit 40. An explanation according to the fourth example will be made with respect to a specific example of the structure of the ultrasound diagnostic apparatus having a plurality of optimal positions for elastic modulus measurement output from the measurement position selecting unit 40.

The system according to the fourth example is configured to allow the measurement position selecting unit 40 to output all the positions P (x,y) of the kernel K, at which the standard deviation S (x,y) of the strain is equal to or smaller than the threshold value upon selection of the elastic modulus measurement position by producing the kernel K. When producing the kernel K and calculating the difference between the maximum and minimum values of the strain amount, the measurement position selecting unit 40 outputs all the positions P (x,y) of the kernel K, at which the difference in the strain amount between the maximum and minimum values, that is, Max-min(x,y) is equal to or smaller than the threshold value. When executing the image processing by producing the filter G, the measurement position selecting unit 40 outputs all the regions R(n) larger than the filter G. The threshold value is read from the not shown storage medium by the central control unit 3, or input by the operator via the not shown recording medium.

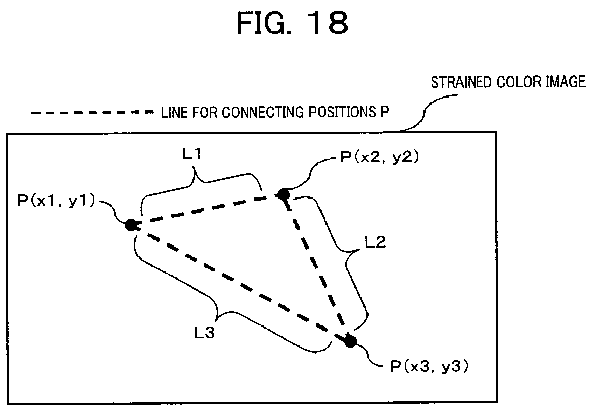

As FIG. 18 shows, the system having three positions P output from the measurement position selecting unit 40, that is, P(x1,y1), P(x2,y2), and P(x3,y3) will be described. The number of the positions P output from the measurement position selecting unit 40 is not limited to 3, but may be an integer equal to or larger than 2. In the fourth example, the measurement position selecting unit 40 calculates each distance between adjacent positions P, that is, L1, L2 and L3. The distance information is output to the central control unit 3 together with the position information. The central control unit 3 outputs the focal point positions F corresponding to the three positions P to the focal point position setting unit 12. At this time, the order of the focal point positions output from the central control unit 3 is controlled in accordance with the distance between the focal points. An example of FIG. 18 shows the order of L3>L2>L1. The central control unit 3 determines the output order of the three focal point positions F so that the distance between the two focal point positions F output at the time points in succession is in the order from the long to the short distance. The hybrid method according to the fourth example measures the elastic modulus at each one of those three focused positions, and updates the elastic modulus image for each performance of the elastic modulus measurement.

The processing flow of the hybrid method according to the fourth example will be described referring to the example shown in FIG. 18. The description will be made with respect to application to the first example. The different part from that of the first example will only be described hereinafter. The focal points F corresponding to the positions P(x1,y1), P(x2,y2), and P(x3,y3) are determined as F(x1-$\Delta$x,y1), F(x2-$\Delta$x,y2), and F(x3-$\Delta$x,y3), respectively.

The aforementioned three positions are selected in step S06 shown in FIG. 3, and the distance is computed. Then in step S08 shown in FIG. 3, the focal point F(x1-$\Delta$x,y1) is output from the central control unit 3 to the focal point position setting unit 12. The focused beam as the displacement generating transmission beam is radiated to the focal point F(x1-$\Delta$x,y1) for measuring the elastic modulus. After displaying the elastic modulus image in step S10, if the measurement is not finished in step S12, the process returns to step S08. Then the F(x3-$\Delta$x,y3) is output from the central control unit 3 to the focal point position setting unit 12 so that the focused beam is radiated to the focal point F(x3-$\Delta$x,y3) so as to measure the elastic modulus.

After displaying the elastic modulus image in step S10, if the measurement is not finished in step S12, the process returns to step S08. Then the F(x2-$\Delta$x,y2) is output from the central control unit 3 to the focal point position setting unit 12. The focused beam is radiated to the focal point F(x2-$\Delta$x,y2) so as to measure the elastic modulus. After displaying the elastic modulus image in step S10, if the measurement is not finished in step S12, the process returns to step S02 so as to measure the strain distribution. Then in step S06, a plurality of elastic modulus measurement positions are selected. At this time, instead of selecting a plurality of elastic modulus measurement positions again, three positions selected at the previous time may be used.

In this way, a plurality of elastic modulus measurement positions is selected in step S06 to ensure reduction in time for calculation and measurement. At the two elastic modulus measurement positions at the time points in succession for measuring the elastic modulus, the position at a distance from the focal point position irradiated at the previous time is selected as the second or subsequent position. Accordingly, this makes it possible to minimize the influence of the temperature rise generated by irradiating the focal point position corresponding to the respective positions with the focused beam to the temperature rise at the other focal point position. The focused beam is not radiated to the same location consecutively, making it possible to reduce the local heat temperature rise.

The aforementioned description relates to the application to the first example. Likewise, the output order of the focal point positions is controlled in the second and the third examples. Fifth Example

A fifth example describes another type of the elastic modulus measurement position selection method as described in the first to the third examples. The explanation will be made herein on the assumption that the system configuration according to the first example is employed, and each size of the kernel K and the filter G is the same as the region ROI_e where the elastic modulus is measured.

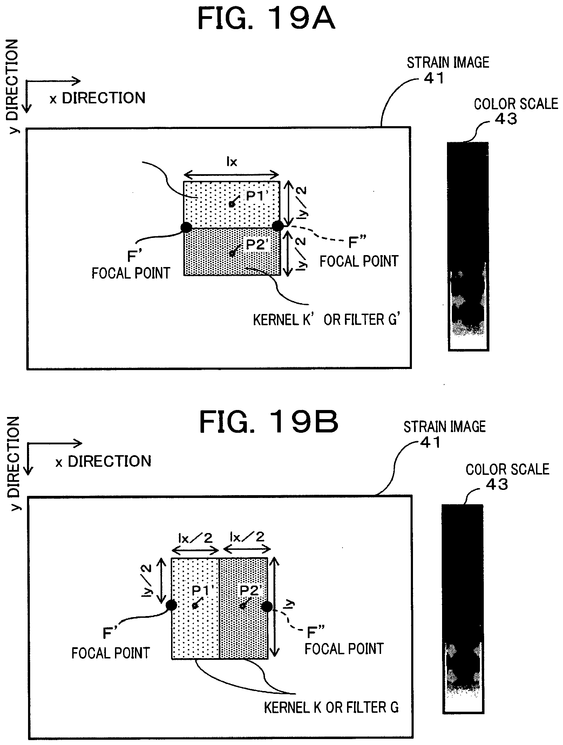

According to the fifth example, the kernel K or the filter G has the size reduced by half in the y direction as FIG. 19A shows, and the signal processing is executed for selecting the elastic modulus measuring position. In this example, a +y direction denotes a propagation direction of the focused beam as the displacement generating transmission beam, and +x direction denotes the shear wave propagation direction. As described above, the length of the ROI_e in the y direction is determined by the width of the displacement generating beam in the depth direction. The kernel K or filter G having the size reduced by half in the y direction will be referred to as the kernel K' or filter G', respectively. If the length of the kernel K or the filter G in the depth direction is set to ly, and the length in the orientation direction is set to lx, the length of the kernel K' or the filter G' in the depth direction is ly/2, and the length in the orientation direction is lx. Preferably, the length in the depth direction is the value half the width of the displacement generating transmission beam in the depth direction.

When producing the kernel K' for calculation, all the positions P'(x,y) of the kernel K', at which the strain standard deviation S'(x,y) is equal to or smaller than the threshold value are derived from calculation. When producing the kernel K' and calculating the difference between the maximum and minimum values of the strain amount, all the positions P'(x,y) of the kernel K', at which the difference between the maximum and minimum values Max-min(x,y) of the strain amount is equal to or smaller than the threshold value are derived from calculation. When producing the filter G' for image processing, all the regions R'(n)(n: positive integer) larger than the filter G', and the positions P'(x,y) each as center of gravity of the R'(n) are derived from the image processing. The threshold value is read by the central control unit 3 from the not shown storage medium, and automatically set, or input by the operator via the not shown storage medium and manually set.

After the signal processing using the kernel K' or the filter G' and calculating the elastic modulus measurement positions P'(x,y), the measurement position selecting unit 40 searches two positions P'(x,y) at which the kernels K' or the filters G' are in succession. For example, two positions P' (x,y) include a position P1'(x',y') and the other position P2'(x',y'+ly/2). If the two positions where the kernels K' and the filters G' are in succession in the depth direction are located, the measurement position setting unit 40 outputs the two consecutive position information data P1'(x',y') and P2'(x',y'+ly/2) to the central control unit 3. The central control unit 3 calculates the focal point position F' based on the positions P1' and P2'.

As FIG. 19A shows, an x-coordinate of the F' is x' - lx/2, and a y-coordinate is y'+ly/4. The lx/2 is the value corresponding to the distance half the shear wave propagation distance. The y'+ly/4 is the value corresponding to the center of the y-coordinates between the positions P1' and P2', that is, the value corresponding to half the length of the region ROI_e for measuring the elastic modulus in the depth direction. The information of the focal point F' is input to the focal point position setting unit 12, and the displacement generating transmission beam is radiated to the focal point F'. The displacement of the shear wave propagating from the focal point F' in the x direction is detected.

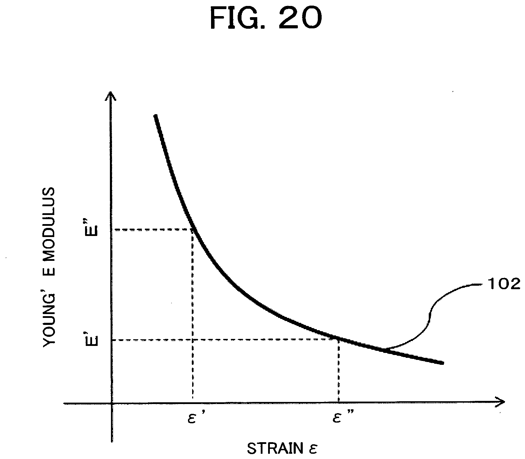

when calculating the elastic modulus in the ROI_e, the elastic modulus computing unit 34 calculates two values of the elastic modulus, that is, the one at the location shallower than the focal point F' and the other at the location deeper than the focal point F'. In the fifth example, it is possible to obtain the relationship between two values of the strain $\varepsilon'$, $\varepsilon''$ shown in FIG. 20 and the two values of the elastic modulus E', E'' corresponding to those strain values by performing the elastic modulus measurement at the single point. Accordingly, two values of stress are calculated from the two pairs of the strain and elastic modulus. As described above, the color scale setting unit 50 converts the strain color scale into the color scale of the elastic modulus. Clarifying the correlation of two pairs of the strain and elastic modulus makes it possible to estimate the elastic modulus concerning the strain between $\varepsilon'$ and $\varepsilon''$ through interpolation from the E' and E''. If there is no desired strain between $\varepsilon'$ and $\varepsilon''$, the elastic modulus may be obtained through extrapolation. Use of the value obtained by linear interpolation such as the interpolation and extrapolation from the two stresses makes it possible to improve the conversion accuracy. Generally, interpolation results in higher accuracy than the extrapolation. Preferably, the strains $\varepsilon'$ and $\varepsilon''$ have different values so as to improve the estimation accuracy of the elastic modulus over a wide range. For this, the explanation has been made, taking two adjacent regions ROI as the example. However, the regions may be spatially apart from each other so long as the shear wave is propagated to the two ROIs by generating the shear wave once. As described above, if the location at which the shear wave is generated is distant from the measurement point, the signal to noise ratio is deteriorated. In setting of the ROI, the aforementioned two points of view (different strain, signal to noise ratio) have to be considered. One of values of the stress may be calculated by fitting the relationship between the two strain values $\varepsilon'$, $\varepsilon''$ and the two values of elastic modulus E',E'' to the curve expressed by the expression $E=\sigma/\varepsilon$. The fitting process is executed by the color scale setting unit 50 or the central control unit 3. Computation of the fitting is executed by processing the program of the central processing unit. If the two consecutive kernels K' or the filters G' do not exist in the depth direction, each size of the kernel and the filter, or the threshold is automatically or manually changed. If the two consecutive kernels K' or filters G' do not exist in the depth direction, the elastic modulus measurement position is selected by executing the signal processing again using the kernel K and the filter G so as to ensure the elastic modulus measurement using the method according to the first, second and third examples.

The description has been made based on the system according to the first example. However, the system according to the second or the third example may be applied in the similar way. Except reduction in the kernel size by half in the depth direction, such size may be reduced by 1/m (m: positive integer). Upon reduction by 1/m (m: positive integer), the pair of elastic modulus and the strain calculated by the elastic modulus computing unit 34 may be increased by m times. This makes it possible to improve accuracy of the stress calculated through averaging. Upon application of the hybrid method when measuring the elastic modulus, positions of the two focal points are set to $F'(x'-lx/2, y'+ly/4)$ and $F''(x'+lx/2, y'+ly/4)$, respectively.

Each size of the kernel K and the filter G may be reduced by half in the x direction as shown by FIG. 19B, that is, the shear wave propagation direction. At this time, preferably, the length of the shortened kernel K' or the filter G' has the value half the shear wave propagation distance in the orientation direction.

When reducing the size of the kernel K or the filter G by half in the shear wave propagation direction, two values of TM(n) are obtained each as the value of switching frequency Tm reaching the peak with respect to the corresponding n upon measurement of the elastic modulus using the hybrid method. This is because a medium having two different types of the shear wave propagation speeds exists between the two focal points. As the expression 2 indicates, the TM(n) is in inverse proportion to the shear wave propagation speed. The smaller the strain becomes, the higher the shear wave propagation speed is increased. Accordingly, the smaller Tm(n) may be obtained in the region with smaller strain. It is therefore possible to distinguish the strain from the shear wave speed in the two regions, that is, the strain and the elastic modulus.

The test body as the object to be measured in the respective examples includes the living body such as the liver, mammary gland, blood vessel, and prostate gland. The respective examples employ the method of combining the elastic modulus measurement using the shear wave and the color image of strain. As one of features, the strain color image is obtained prior to the elastic modulus measurement. As the strain color image is obtained previously, it is possible to select the location suitable for the elastic modulus measurement. This makes it possible to largely improve accuracy of the elastic modulus measurement as well as accuracy of the combined image based on the elastic modulus measurement.

Various examples of the present invention have been described. However, the present invention is not limited to those examples, and may include various modified examples. The examples have been described for the purpose of facilitating understandings of the present invention which is not limited thereto. A part of the structure of the example may be replaced by the structure of the other example. It is also possible to add the structure of one of the examples to that of the other example. For example, it is possible to combine structures of the fourth and the fifth examples.

In the respective examples, known methods may be used for generating the shear wave instead of the ultrasonic focused beam, for example, mechanical drive (DC motor, oscillating pump), manual compression, compression by way of electric pulse, motion of the tissue of the body such as heart and blood vessel. A two-dimensional probe may be used instead of the probe of linear array type. The respective elements of the ultrasound probe 1 may be changed to ceramic, a piezoelectric device formed of polymer, and the oscillator using electrostatic force of silicon.

The displacement generating transmission beam is radiated to a plurality of positions along its propagation direction so that the planar wave of the shear wave is generated to realize the long shear wave propagation distance. Upon detection of the displacement, the displacement may be calculated using the known calculation method besides the correlation operation, for example, cross-correlation operation, minimum square sum, and Doppler method.

The structure, function and processing unit of the respective examples may be formed as the exclusive hardware structure, software structure, or the structure that shares both the hardware and software.

As various examples have been described so far, various types of the invention may be contained herein except those described in claims.

Reference Signs List

1 ultrasound probe
2 send-receive switch
3 central control unit
4 color DSC
5 black-and-white DSC
6 combining unit
7 display unit
10 displacement generating unit
11 displacement generating transmission waveform generating unit

12 focal point position setting unit

13 displacement generating transmission beam forming unit

14 beam time setting unit

20 first ultrasonic send-receive unit

22 displacement computing unit

24 strain computing unit

30 second ultrasonic send-receive unit

32 shear wave displacement computing unit

34 elastic modulus computing unit

35 stiffness spectrum calculation unit

41 strain image

43 elastic modulus color scale

44 filter G

45 B mode image

46 elastic modulus image

47 elastic modulus (absolute value, maximum-minimum)

40 measurement position selecting unit

50 color scale setting unit

60 elastic modulus measurement position computing unit

61 input unit

62 displacement generating beam propagation path estimation unit

100 elements of ultrasound probe 1

**Claims**

1. An ultrasound diagnostic apparatus using an ultrasound probe (1) for transmitting an ultrasonic beam to a test body and receiving an echo signal, comprising:

   a strain computing unit (24) which is adapted to radiate a first displacement detection beam, and compute strain information in a first region (ROI_s) based on the echo signal from the test body that receives the first displacement detection beam;
   a measurement position selecting unit (40) which is adapted to select a second region (ROI_e) included in the first region (ROI_s) based on the strain information;
   a displacement generating unit (10) which is adapted to radiate a focused beam to an inside of the test body to displace a tissue in the test body; and
   a display unit (7) which is adapted to display a strain image (41),
   **characterized in that**
   the ultrasound diagnostic apparatus further comprises an elastic modulus computing unit (34) which is adapted to radiate a second displacement detection beam, and detect a shear wave displacement generated by the focused beam based on the echo signal from the test body that receives the second displacement detection beam for detecting an elastic modulus in the second region (ROI_e),
   the display unit (7) is adapted to display the strain image (41) based on the strain information and the elastic modulus, and
   the measurement position selecting unit (40) is adapted to obtain a position at which a standard deviation of a strain distribution in the first region (ROI_s) or a difference between a maximum value and a minimum value is smaller than a threshold value when selecting the second region (ROI_e).

2. The ultrasound diagnostic apparatus according to claim 1, wherein
   the measurement position selecting unit (40) is adapted to select at least one elastic modulus detection position at which the elastic modulus is detected based on the strain information, and
   at least one focal point position irradiated by the focused beam is determined from at least the one elastic modulus detection position selected by the measurement position selecting unit (40).

3. The ultrasound diagnostic apparatus according to claim 2, wherein
   the measurement position selecting unit (40) is adapted to select the one elastic modulus detection position at which a strain is uniformly distributed, and
   the two different focal point positions irradiated by the focused beam are determined from the selected elastic modulus detection position.

4. The ultrasound diagnostic apparatus according to claim 3, wherein
the displacement generating unit (10) includes a transmission beam time setting unit (14) which is adapted to set a transmission time of the focused beam, and
the transmission beam time setting unit (14) is adapted to set the transmission time so that the focused beam is radiated to the two different focal point positions at a same ON/OFF switching cycle, while changing the ON/OFF switching cycle to provide a cycle or a phase chirp signal.

5. The ultrasound diagnostic apparatus according to claim 2, wherein
the measurement position selecting unit (40) is adapted to select a plurality of elastic modulus detection positions at which the strain is uniformly distributed, and
at least one of the focal point positions irradiated by the focused beam is determined from the plurality of elastic modulus detection positions selected by the measurement position selecting unit (40).

6. The ultrasound diagnostic apparatus according to claim 2, wherein the measurement position selecting unit (40) is adapted to allow an operator to select the elastic modulus detection position while observing an image displayed on the display unit (7).

7. The ultrasound diagnostic apparatus according to claim 1, wherein the display unit (7) is adapted to display a color scale (43) indicating the elastic modulus in a display range of the strain image (41) to be displayed.

8. The ultrasound diagnostic apparatus according to claim 1, wherein the elastic modulus computing unit (34) is adapted to calculate a stress using the elastic modulus in the second region (ROI_e) and the strain information in the second region (ROI_e), and compute the elastic modulus of the first region (ROI_s) from the strain information in the first region (ROI_s) and the stress.

9. The ultrasound diagnostic apparatus according to claim 1, wherein the measurement position selecting unit (40) is adapted to allow an operator to select the second region (ROI_e) based on the strain image (41) displayed on the display unit (7).

10. An ultrasound display method of displaying an image on a display unit (7) using an ultrasound probe (1) for transmitting an ultrasound beam to a test body and receiving an echo signal from the test body based on the received echo signal, comprising the steps of:

computing strain information in a first region (ROI_s) by radiating a first displacement detection beam and receiving the echo signal from the test body;
displaying a strain image (41) based on the computed strain information on the display unit (7);
displacing a tissue of the test body by radiating a focused beam in the test body;
obtaining a position at which a standard deviation of a strain distribution in the first region (ROI_s) or a difference between a maximum value and a minimum value is smaller than a threshold value when selecting a second region (ROI_e) included in the first region (ROI_s);
radiating the second displacement detection beam and receiving the echo signal from the test body to detect a shear wave displacement generated by the focused beam;
computing an elastic modulus in the second region (ROI_e) based on the shear wave displacement; and
displaying the computed elastic modulus on the display unit (7).

11. The ultrasound display method according to claim 10, wherein a focal point position of the focused beam selected based on the strain information is determined.

12. The ultrasound display method according to claim 10, wherein the second region (ROI_e) irradiated by the second displacement detection beam is selected from a location with the uniform strain information based on the strain image (41) displayed on the display unit (7).

13. The ultrasound display method according to claim 10, wherein the display unit (7) displays a scale (43) indicating the elastic modulus in a display range of the strain image (41) to be displaced.

**EP 2 671 511 B1**

**Patentansprüche**

1.  Ultraschalldiagnosegerät, das eine Ultraschallsonde (1) zum Senden eines Ultraschallstrahls auf ein Testobjekt und Empfangen eines Echosignals verwendet, mit

    einer Verformungsberechnungseinheit (24), die dazu ausgelegt ist, einen ersten Verschiebungserfassungsstrahl auszustrahlen und Verformungsinformation in einer ersten Region (ROI_s) basierend auf dem Echosignal aus dem Testkörper zu berechnen, der den ersten Verschiebungserfassungsstrahl empfängt,

    einer Messpositionauswähleinheit (40), die dazu ausgelegt ist, eine zweite, in der ersten Region (ROI_s) enthaltene Region (ROI_e) basierend auf der Verformungsinformation auszuwählen,

    einer Verschiebungserzeugungseinheit (10), die dazu ausgelegt ist, einen fokussierten Strahl auf ein Inneres des Testkörpers auszustrahlen, um ein Gewebe in dem Testkörper zu verschieben, und

    einer Anzeigeeinheit (7), die dazu ausgelegt ist, ein Verformungsbild (41) anzuzeigen,

    **dadurch gekennzeichnet, dass**

    das Ultraschalldiagnosegerät ferner eine Elastizitätsmodul-Berechnungseinheit (34) aufweist, die dazu ausgelegt ist, einen zweiten Verschiebungserfassungsstrahl auszustrahlen und eine durch den fokussierten Strahl erzeugte Scherwellenverschiebung basierend auf dem Echosignal aus dem Testkörper zu erfassen, der den zweiten Verschiebungserfassungsstrahl zum Erfassen eines Elastizitätsmoduls in der zweiten Region (ROI_e) empfängt,

    die Anzeigeeinheit (7) dazu ausgelegt ist, das Verformungsbild (41) basierend auf der Verformungsinformation und dem Elastizitätsmodul anzuzeigen, und

    die Messpositionauswähleinheit (40) dazu ausgelegt ist, eine Position zu erhalten, an der eine Standardabweichung einer Verformungsverteilung in der ersten Region (ROI_s) oder eine Differenz zwischen einem Maximalwert und einem Minimalwert kleiner ist als ein Schwellenwert, wenn die zweite Region (ROI_e) ausgewählt ist.

2.  Ultraschalldiagnosegerät nach Anspruch 1, wobei

    die Messpositionauswähleinheit (40) dazu ausgelegt ist, basierend auf der Verformungsinformation wenigstens eine Elastizitätsmodul-Erfassungsposition auszuwählen, an der das Elastizitätsmodul erfasst wird, und

    wenigstens eine durch den fokussierten Strahl bestrahlte Fokalpunktposition aus wenigstens der einen Elastizitätsmodul-Erfassungsposition bestimmt wird, die durch die Messpositionauswähleinheit (40) ausgewählt wurde.

3.  Ultraschalldiagnosegerät nach Anspruch 2, wobei

    die Messpositionauswähleinheit (40) dazu ausgelegt ist, die eine Elastizitätsmodul-Erfassungsposition auszuwählen, an der eine Verformung gleichmäßig verteilt ist, und

    die zwei verschiedenen, durch den fokussierten Strahl bestrahlten Fokalpunktpositionen aus der ausgewählten Elastizitätsmodul-Erfassungsposition bestimmt werden.

4.  Ultraschalldiagnosegerät nach Anspruch 3, wobei

    die Verschiebungserzeugungseinheit (10) eine Sendestrahl-Zeiteinstelleinheit (14) aufweist, die dazu ausgelegt ist, eine Sendezeit des fokussierten Strahls einzustellen, und

    die Sendestrahl-Zeiteinstelleinheit (14) dazu ausgelegt ist, die Sendezeit so einzustellen, dass der fokussierte Strahl zu den zwei verschiedenen Fokalpunktpositionen zu einem gleichen AN/AUS-Umschaltzyklus ausgestrahlt wird, während der AN/AUS-Umschaltzyklus geändert wird, um ein Takt- oder Phasen-Chirp-Signal bereitzustellen.

5.  Ultraschalldiagnosegerät nach Anspruch 2, wobei

    die Messpositionauswähleinheit (40) dazu ausgelegt ist, mehrere Elastizitätsmodul-Erfassungspositionen auszuwählen, an denen die Verformung gleichmäßig verteilt ist, und

    wenigstens eine der durch den fokussierten Strahl bestrahlten Fokalpunktpositionen aus den mehreren Elastizitätsmodul-Erfassungspositionen bestimmt wird, die durch die Messpositionauswähleinheit (40) ausgewählt wurden.

6.  Ultraschalldiagnosegerät nach Anspruch 2, wobei die Messpositionauswähleinheit (40) dazu ausgelegt ist, einem Bediener zu erlauben, die Elastizitätsmodul-Erfassungsposition auszuwählen, während ein auf der Anzeigeeinheit (7) angezeigtes Bild beobachtet wird.

7.  Ultraschalldiagnosegerät nach Anspruch 1, wobei die Anzeigeeinheit (7) dazu ausgelegt ist, eine Farbskala (43) anzuzeigen, die das Elastizitätsmodul in einem Anzeigebereich des anzuzeigenden Verformungsbilds (41) angibt.

8.  Ultraschalldiagnosegerät nach Anspruch 1, wobei die Elastizitätsmodul-Berechnungseinheit (34) dazu ausgelegt ist, eine Spannung unter Verwendung des Elastizitätsmoduls in der zweiten Region (ROI_e) und der Verformungsinformation in der zweiten Region (ROI_e) zu berechnen, und das Elastizitätsmodul der ersten Region (ROI_s) aus

**22**

der Verformungsinformation in der ersten Region (ROI_s) und der Spannung zu berechnen.

9. Ultraschalldiagnosegerät nach Anspruch 1, wobei die Messpositionauswähleinheit (40) dazu ausgelegt ist, einem Bediener zu erlauben, die zweite Region (ROI_e) basierend auf dem auf der Anzeigeeinheit (7) angezeigten Verformungsbild (41) auszuwählen.

10. Ultraschalldiagnoseverfahren um, unter Verwendung einer Ultraschallsonde (1) zum Senden eines Ultraschallstrahls auf einen Testkörper und zum Empfangen eines Echosignals aus dem Testkörper, basierend auf dem empfangenen Echosignal ein Bild auf einer Anzeigeeinheit (7) anzuzeigen, wobei in den Schritten des Verfahrens:

   Verformungsinformation in einer ersten Region (ROI_s) berechnet wird, indem ein erster Verschiebungserfassungsstrahl ausgestrahlt und das Echosignal aus dem Testkörper empfangen wird,
   basierend auf der berechneten Verformungsinformation ein Verformungsbild (41) auf der Anzeigeeinheit (7) angezeigt wird,
   ein Gewebe des Testkörpers verschoben wird, indem ein fokussierter Strahl in den Testkörper ausgestrahlt wird,
   eine Position erhalten wird, an der eine Standardabweichung einer Verschiebungsverteilung in der ersten Region (ROI_s) oder eine Differenz zwischen einem Maximalwert und einem Minimalwert kleiner ist als ein Schwellenwert, wenn eine in der ersten Region (ROI_s) enthaltene zweite Region (ROI_e) ausgewählt ist,
   der zweite Verschiebungserfassungsstrahl ausgestrahlt wird und das Echosignal aus dem Testkörper empfangen wird, um eine durch den fokussierten Strahl erzeugte Scherwellenverschiebung zu erfassen,
   basierend auf der Scherwellenverschiebung ein Elastizitätsmodul in der zweiten Region (ROI_e) berechnet wird, und
   das berechnete Elastizitätsmodul auf der Anzeigeeinheit (7) angezeigt wird.

11. Ultraschallanzeigeverfahren nach Anspruch 10, wobei eine Fokalpunktposition des basierend auf der Verformungsinformation ausgewählten fokussierten Strahls bestimmt wird.

12. Ultraschallanzeigeverfahren nach Anspruch 10, wobei die durch den zweiten Verschiebungserfassungsstrahl bestrahlte Region (ROI_e) basierend auf dem auf der Anzeigeeinheit (7) angezeigten Verformungsbild (41) aus einem Ort mit der gleichmäßigen Verformungsinformation ausgewählt wird.

13. Ultraschallanzeigeverfahren nach Anspruch 10, wobei die Anzeigeeinheit (7) eine Skala (43) anzeigt, die das Elastizitätsmodul in einem Anzeigebereich des zu verschiebenden Verformungsbilds (41) angibt.

**Revendications**

1. Appareil de diagnostic par ultrasons utilisant une sonde (1) à ultrasons pour transmettre un faisceau ultrasonore à un corps de test et recevoir un signal d'écho, comprenant :

   une unité (24) de calcul de déformation qui est adaptée à projeter un premier faisceau de détection de déplacement, et calculer une information de déformation dans une première région (ROI_s) sur la base du signal d'écho provenant du corps de test qui reçoit le premier faisceau de détection de déplacement ;
   une unité (40) de sélection de position de mesure qui est adaptée à sélectionner une deuxième région (ROI_e) incluse dans la première région (ROI_s) sur la base de l'information de déformation ;
   une unité (10) de génération de déplacement qui est adaptée à projeter un faisceau focalisé jusqu'à un intérieur du corps de test pour déplacer un tissu dans le corps de test ; et
   une unité (7) d'affichage qui est adaptée à afficher une image (41) de déformation,
   **caractérisé en ce que**
   l'appareil de diagnostic par ultrasons comprend en outre une unité (34) de calcul de module d'élasticité qui est adaptée à projeter un deuxième faisceau de détection de déplacement, et détecter un déplacement d'onde de cisaillement généré par le faisceau focalisé sur la base du signal d'écho provenant du corps de test qui reçoit le deuxième faisceau de détection de déplacement pour détecter un module d'élasticité dans la deuxième région (ROI_e),
   l'unité (7) d'affichage est adaptée à afficher l'image (41) de déformation sur la base de l'information de déformation et du module d'élasticité, et
   l'unité (40) de sélection de position de mesure est adaptée à obtenir une position à laquelle un écart type d'une distribution de déformation dans la première région (ROI_s) ou une différence entre une valeur maximum et une valeur minimum est plus petit(e) qu'une valeur de seuil lors de la sélection de la deuxième région (ROI_e).

2. Appareil de diagnostic par ultrasons selon la revendication 1, dans lequel
l'unité (40) de sélection de position de mesure est adaptée à sélectionner au moins une position de détection de module d'élasticité à laquelle le module d'élasticité est détecté sur la base de l'information de déformation, et au moins une position de point focal irradiée par le faisceau focalisé est déterminée à partir d'au moins la position de détection de module d'élasticité sélectionnée par l'unité (40) de sélection de position de mesure.

3. Appareil de diagnostic par ultrasons selon la revendication 2, dans lequel
l'unité (40) de sélection de position de mesure est adaptée à sélectionner la position de détection de module d'élasticité à laquelle une déformation est uniformément distribuée, et les deux positions de point focal différentes irradiées par le faisceau focalisé sont déterminées à partir de la position de détection de module d'élasticité sélectionnée.

4. Appareil de diagnostic par ultrasons selon la revendication 3, dans lequel
l'unité (10) de génération de déplacement inclut une unité (14) de réglage de durée de faisceau de transmission qui est adaptée à régler une durée de transmission du faisceau focalisé, et l'unité (14) de réglage de durée de faisceau de transmission est adaptée à régler la durée de transmission de telle manière que le faisceau focalisé est projeté sur les deux positions de point focal différentes à un même cycle de commutation activation/désactivation, tout en changeant le cycle de commutation activation/désactivation pour donner un signal chirp de cycle ou de phase.

5. Appareil de diagnostic par ultrasons selon la revendication 2, dans lequel
l'unité (40) de sélection de position de mesure est adaptée à sélectionner une pluralité de positions de détection de module d'élasticité auxquelles la déformation est uniformément distribuée, et au moins une des positions de point focal irradiées par le faisceau focalisé est déterminée à partir de la pluralité de positions de détection de module d'élasticité sélectionnée par l'unité (40) de sélection de position de mesure.

6. Appareil de diagnostic par ultrasons selon la revendication 2, dans lequel l'unité (40) de sélection de position de mesure est adaptée à permettre à un opérateur de sélectionner la position de détection de module d'élasticité tout en observant une image affichée sur l'unité (7) d'affichage.

7. Appareil de diagnostic par ultrasons selon la revendication 1, dans lequel l'unité (7) d'affichage est adaptée à afficher une échelle (43) de couleurs indiquant le module d'élasticité dans une plage d'affichage de l'image (41) de déformation devant être affichée.

8. Appareil de diagnostic par ultrasons selon la revendication 1, dans lequel l'unité (34) de calcul de module d'élasticité est adaptée à calculer une contrainte en utilisant le module d'élasticité dans la deuxième région (ROI_e) et l'information de déformation dans la deuxième région (ROI_e), et calculer le module d'élasticité de la première région (ROI_s) à partir de l'information de déformation dans la première région (ROI_s) et de la contrainte.

9. Appareil de diagnostic par ultrasons selon la revendication 1, dans lequel l'unité (40) de sélection de position de mesure est adaptée à permettre à un opérateur de sélectionner la deuxième région (ROI_e) sur la base de l'image (41) de déformation affichée sur l'unité (7) d'affichage.

10. Procédé d'affichage par ultrasons pour afficher une image sur une unité (7) d'affichage en utilisant une sonde (1) à ultrasons pour transmettre un faisceau d'ultrasons à un corps de test et recevoir un signal d'écho provenant du corps de test sur la base du signal d'écho reçu, comprenant les étapes de :

  calcul d'une information de déformation dans une première région (ROI_s) en projetant un premier faisceau de détection de déplacement et en recevant le signal d'écho provenant du corps de test ;
  affichage d'une image (41) de déformation sur la base de l'information de déformation calculée sur l'unité (7) d'affichage ;
  déplacement d'un tissu du corps de test en projetant un faisceau focalisé dans le corps de test ;
  obtention d'une position à laquelle un écart type d'une distribution de déformation dans la première région (ROI_s) ou une différence entre une valeur maximum et une valeur minimum est plus petit(e) qu'une valeur de seuil lors de la sélection d'une deuxième région (ROI_e) incluse dans la première région (ROI_s) ;
  projection du deuxième faisceau de détection de déplacement et réception du signal d'écho provenant du corps de test pour détecter un déplacement d'onde de cisaillement généré par le faisceau focalisé ;

calcul d'un module d'élasticité dans la deuxième région (ROI_e) sur la base du déplacement d'onde de cisaillement ; et

affichage du module d'élasticité calculé sur l'unité (7) d'affichage.

11. Procédé d'affichage par ultrasons selon la revendication 10, dans lequel une position de point focal du faisceau focalisé sélectionnée sur la base de l'information de déformation est déterminée.

12. Procédé d'affichage par ultrasons selon la revendication 10, dans lequel la deuxième région (ROI_e) irradiée par le deuxième faisceau de détection de déplacement est sélectionnée à partir d'un emplacement avec l'information de déformation uniforme sur la base de l'image (41) de déformation affichée sur l'unité (7) d'affichage.

13. Procédé d'affichage par ultrasons selon la revendication 10, dans lequel l'unité (7) d'affichage affiche une échelle (43) indiquant le module d'élasticité dans une plage d'affichage de l'image (41) de déformation devant être affichée.

# FIG. 1

# FIG. 2

DISPLAY UNIT
7

B MODE IMAGE
45

STRAIN IMAGE
41

ROI_e(1)    ROI_e(2)

hard

COLOR SCALE
43

soft

ROI_s

# FIG. 3

START DIAGNOSIS — S00

MEASURE STRAIN DISTRIBUTION — S02

DISPLAY STRAIN IMAGE — S04

SELECT ELASTIC MODULUS MEASUREMENT POSITION — S06

MEASURE ELASTIC MODULUS — S08

DISPLAY HYBRID IMAGE — S10

IS DIAGNOSIS FINISHED? — S12

NO

YES

END — S14

# FIG. 4A

P(x, y)  KERNEL K  42  STRAIN IMAGE  41

x KERNEL MOVING DIRECTION

y KERNEL MOVING DIRECTION

COLOR SCALE  43

lx

ly

F(x1+Δx, y1)

F(x1-Δx, y1)  P(x1, y1)

# FIG. 4B

lx

44 FILTER G  41

ly

FILTER G

REGION (1)

REGION (2)

REGION (3)

## FIG. 5A

DRIVING PULSE

TIME

100

FOCAL POINT

## FIG. 5B

DRIVING PULSE

TIME

100

FOCAL POINT

# FIG. 6

BODY SURFACE

1

100

SHEAR WAVE DISPLACEMENT DIRECTION

SHEAR WAVE DISPLACEMENT DIRECTION

SHEAR WAVE DISPLACEMENT DIRECTION

SHEAR WAVE DISPLACEMENT DIRECTION

FOCAL POINT F

# FIG. 7

# FIG. 8

# FIG. 9

DISPLAY UNIT
7

B MODE IMAGE
45

ELASTIC MODULUS IMAGE
46

47
20kPa

COLOR SCALE
43

47
0kPa

ROI_s

# FIG. 10

# FIG. 11

BODY SURFACE

DISPLACEMENT DIRECTION

SHEAR WAVE DISPLACEMENT DIRECTION

F1

FOCAL POINT

F2

DISPLACEMENT DIRECTION

SHEAR WAVE DISPLACEMENT DIRECTION

d

1

100

## FIG. 12

# FIG. 13

ABSOLUTE VALUE OF SHEAR WAVE
DISPLACEMENT AMOUNT

103

π    3π    5π    7π    kd

# FIG. 14

SHEAR WAVE
PROPAGATION DIRECTION

SHEAR WAVE
PROPAGATION DIRECTION

SHEAR WAVE
DISPLACEMENT DIRECTION

F1    F2

MEASUREMENT POINT A    MEASUREMENT POINT B

# FIG. 15

# FIG. 16A

SIGNAL WAVEFORM OF SHEAR WAVE
GENERATED FROM EACH FOCAL POINT

AMPLITUDE

# FIG. 16B

SIGNAL WAVEFORM OF
INTERFERED SHEAR WAVE

# FIG. 16C

SIGNAL WAVEFORM OF SHEAR WAVE
GENERATED FROM EACH FOCAL POINT

AMPLITUDE

# FIG. 16D

SIGNAL WAVEFORM OF
INTERFERED SHEAR WAVE

# FIG. 17

# FIG. 18

# FIG. 19A

# FIG. 19B

# FIG. 20

# FIG. 21

## FIG. 22A

DISPLAY UNIT
7

45
B MODE IMAGE

46
ELASTIC MODULUS IMAGE

43
COLOR SCALE

FOCAL POINT OF
DISPLACEMENT-
GENERATING BEAM

ESTIMATED
DISPLACEMENT-
GENERATING BEAM

ROI_s

## FIG. 22B

REGION LIKE BONE PREFERRED TO BE
KEPT FROM STRONG ULTRASONIC IRRADIATION

45
B MODE IMAGE

DISPLAY UNIT
7

46
ELASTIC MODULUS IMAGE

43
COLOR SCALE

ROI_s

EP 2 671 511 B1

# FIG. 23

46

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004068184 A
- JP 2009201989 A
- US 2009216119 A1
- US 2010016718 A1

**Non-patent literature cited in the description**

- **H. YOSHIKAWA et al.** *Japanese Journal of Applied Physics,* 2006, vol. 45 (5B), 4754